# EUROPEAN PATENT SPECIFICATION

(11) **EP 3 194 418 B1**
(45) Date of publication and mention of the grant of the patent: **15.12.2021**
(21) Application number: 15841632.1
(22) Date of filing: 17.09.2015
(51) Int. Cl.: C07J 15/00, C07B 61/00, C07B 41/04, C07J 61/00, C07J 75/00, C07F 9/50, C07F 9/655, C07F 7/08, C07F 7/18

(54) **SYNTHESIS OF ENT-PROGESTERONE AND INTERMEDIATES THEREOF**
SYNTHESE VON ENT-PROGESTERON UND ZWISCHENPRODUKTE DAVON
SYNTHÈSE DE L'ÉNANTIOMÈRE DE LA PROGESTÉRONE ET SES INTERMÉDIAIRES

(30) Priority: 17.09.2014 US 201462051900 P
(43) Date of publication of application: 26.07.2017
(73) Proprietor: Prevacus Inc., Tallahassee, FL 32308 (US); The Florida State University Research Foundation, Incorporated, Tallahassee, FL 32310 (US)
(72) Inventor: LEVY, Daniel E., San Mateo, CA 94402 (US); CRAN, John W., Tallahassee, FL 32301 (US)
(74) Representative: Loo, Chi Ching
(86) International application number: PCT/US2015/050631
(87) International publication number: WO 2016/044558

(56) References cited:
- WO-A2-2010/088409
- WO-A2-2014/145302
- US-A- 2 623 071
- US-A- 4 189 431
- WILLIAM S. JOHNSON ET AL: "Acetylenic bond participation in biogenetic-like olefinic cyclizations. II. Synthesis of dl-progesterone", JOURNAL OF THE AMERICAN CHEMICAL SOCIETY, vol. 93, no. 17, 1 August 1971 (1971-08-01), pages 4332-4334, XP55519930, ISSN: 0002-7863, DOI: 10.1021/ja00746a062
- MICHAEL B. GRAVESTOCK ET AL: "Acetylenic bond participation in biomimetic polyene cyclizations. Model studies directed toward the synthesis of 20-keto steroids. Synthesis of dl-progesterone and dl-.DELTA.4-androstene-3,17-dione", JOURNAL OF THE AMERICAN CHEMICAL SOCIETY, vol. 100, no. 13, 26 September 1978 (1978-09-26), pages 4274-4282, XP55519912, ISSN: 0002-7863, DOI: 10.1021/ja00481a044
- MOLANDER, G. A. ET AL.: 'Thermal rearrangements of alpha-(omega-azidoalkyl) enones' TETRAHEDRON LETTERS vol. 43, no. 31, 2002, pages 5385 - 5388, XP004370265
- JIANG, C. ET AL.: 'Efficient synthesis of conjugated alkynyl cycloalkenones : Pd (PPh3) 4-AgOAc-catalyzed direct coupling of 1?alkynes with 3-oxocycloal kenyl triflates' APPLIED ORGANOMETALLIC CHEMISTRY vol. 24, no. 3, 2010, pages 208 - 214, XP055418867
- None

## Description

### Field of the Invention

The present invention relates to the method for preparing ent-progesterone.

### Background

Progesterone Is a C-21 steroid hormone involved in the female menstrual cycle, pregnancy and embryogenesis of humans and other species. Progesterone belongs to a class of hormones called progestogens, and is the major naturally occurring human progestogen.

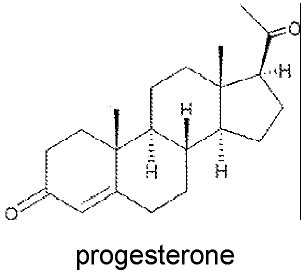

Progesterone is naturally produced by the ovaries of mammals, but may also be produced by some plants and yeast. An economical semi-synthesis of progesterone from the plant steroid diosgenin isolated from yams was developed by Russell Marker in 1940 for the Parke-Davis pharmaceutical company [Marker RE, Krueger J (1940). "Sterols. CXII. Sapogenins. XLI. The Preparation of Trillin and its Conversion to Progesterone". J. Am. Chem. Soc. 62 (12): 3349-3350]. This synthesis is known as the Marker degradation. Additional semi-syntheses of progesterone have also been reported starting from a variety of steroids. For the example, cortisone may be simultaneously deoxygenated at the C-17 and C-21 position by treatment with iodotrimethylsilane in chloroform to produce 11-keto-progesterone (ketogestin), which in turn may be reduced at position-11 to yield progesterone. [Numazawa M, Nagaoka M, Kunitama Y (September 1986). "Regiospecific deoxygenation of the dihydroxyacetone moiety at C-17 of corticoid steroids with iodotrimethylsilane". Chem. Pharm. Bull. 34 (9): 3722-6].

A total synthesis of progesterone was reported in 1971 by W.S. Johnson. [Johnson WS, Gravestock MB, McCarry BE (August 1971). "Acetylenic bond participation in biogenetic-like olefinic cyclizations. II. Synthesis of dl-progesterone". J. Am. Chem. Soc. 93 (17): 4332-4].

The use of progesterone and its analogues have many medical applications, both to address acute situations and to address the long-term decline of natural progesterone levels. Other uses of progesterone include the prevention of preterm birth, to control anovulatury bleeding, to increase skin elasticity and bone strength, and to treat multiple sclerosis.

Progesterone is also useful for the treatment of traumatic brain injury: it reduces poor outcomes following injury by inhibiting inflammatory factors (TNF-a and IL-113) and subsequently reducing brain edema (Pan, D., et al. (2007), Biomed Environ Sci 20, 432-438; Jiang, C., et al. (2009), Inflamm Res 58, 619-624.) Progesterone-treated rats have demonstrated significant improvements on a Neurological Severity Score (test for motor and cognitive functioning) following injury (Roof, R. L., et al. (1992) , Restor Neurol Neurosci 4, 425-427). Progesterone effectively attenuates edema in both rodent sexes following injury (Djebaili, M., et al. (2005), J Neurotrauma 22, 106-118)). Administering Progesterone or its derivative allopregnanolone (ALLO) also results in a decreased of the presence of the factors of cell death (caspase-3) and gliosis (GFAP) (Cutler, S. M., et al. (2007), J Neurotrauma 24, 1475-1486) following injury (VanLandingham, J. W., et al. (2007), Neurosci Lett 425, 94-98; Wright, D. W., et al. (2007), Ann Emerg Med 49, 391-402, 402 e391-392 ). See also, Progesterone for the Treatment of Traumatic Brain Injury (ProTECT III), ClinicalTrials.gov Identifier:NCT00822900; Efficacy and Safety Study of Intravenous Progesterone in Patients With Severe Traumatic Brain Injury (SyNAPSe), ClinicalTrials.gov Identifier:NCT01143064; Progesterone Treatment of Blunt Traumatic Brain Injury, ClinicalTrials.gov Identifier:NCT00048646; and Blood Tests to Study Injury Severity and Outcome in Traumatic Brain Injury Patients (BioProTECT), ClinicalTrials.gov Identifier:NCT01730443. See further, ProTECTTmIII, Progesterone for the Treatment of Traumatic Brain Injury at http://sitemaker.umich.edu/protect/home; Progesterone for Traumatic Brain Injury Tested in Phase III Clinical Trial at http://www.sciencedaily.com/releases/2010/02/100219204407.htm; BHR Pharma Investigational Traumatic Brain Injury Treatment Receives European Medicines Agency Orphan Medicinal Product Designation at http://finance.yahoo.com/news/bhr-pharma-investigational-traumatic-brain-151600948.html; and BHR Pharma SyNAPSe^{®} Trial DSMB Data Analyses Determine No Safety Issues; Study Should Continue to Conclusion at http://www.prnewswire.com/news-releases/bhr-pharma-synapse-trial-dsmb-data-analyses-determine-no-safety-issues-study-should-continue-to-conclusion-187277871.html.

Progesterone exists in a non-naturally occurring enantiomeric form known as ent-progesterone.

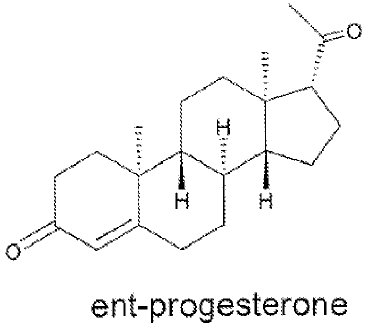

Ent-Progesterone has been shown to have equal efficacy to progesterone in reducing cell death, brain swelling, and inflammation. In addition, ent-progesterone has three times the antioxidant activity of progesterone. Similarly, ent-progesterone has been found to have fewer sexual side effects such as suppression of spermatogenesis; inhibition of the conversion of testosterone to dihydrotestosterone; reduction in the size of the testes, epididymis, and Leydig cells; and no hyper-coagulative risk as may be seen with progesterone. In addition, utilities for ent-progesterone have been described in U.S. Patent Application No. 13/645,881, which was filed on October 5, 2012 and is entitled "Nasal Delivery Mechanism for Prophylactic and Post-Acute Use for Progesterone and/or Its Enantiomer for Use in Treatment of Mild Traumatic Brain Injuries, U.S. Patent Application No. 13/645,854, which was filed on October 12, 2012 and is entitled "Prophylactic and Post-Acute Use of Progesterone and Its Enantiomer to Better Outcomes Associated with Concussion," and U.S. Patent Application No. 13/645,925, which was filed on October 12, 2012 and is entitled "Prophylactic and Post-15 Acute Use of Progesterone in Conjunction with Its Enantiomer for Use in Treatment of Traumatic Brain Injuries. See also VanLandingham et al., Neuropharmacology, The enantiomer of progesterone acts as a molecular neuroprotectant after traumatic brain injury, 2006, 51, 1078- 1085.

Nevertheless, it has been difficult to synthesize ent-progesterone. Previous attempts to synthesize ent-progesterone have suffered from such difficulties as: poor yields, hazardous conditions, hazardous reaction steps, numerous reaction steps and costly reaction steps. These difficulties in synthesizing ent-progesterone have made the commercial use of ent-progesterone and the scale-up of ent-progesterone production unfeasible. WO2010/088409 discloses compositions and methods for treating or preventing nervous system injury using ent-progesterone and also discloses the synthesis of this compound.

As such, there exists for a need for an efficient synthesis of ent-progesterone.

### Summary of the Invention

In one aspect, the invention provides a method for preparing ent-progesterone comprising the step of reacting a compound of the formula U: 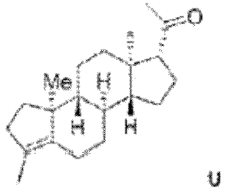 to form a triketone intermediate of the formula **U'** 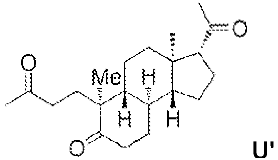 via the steps of:
contacting the compound of formula U with a dihydroxylating reagent which is an osmium reagent or a manganese reagent, thereby forming a vicinal diol, and,
oxidatively cleaving the vicinal diol, thereby forming the compound of formula **U'**,
followed by cyclization of the compound of formula **U'** (e.g., in the presence of base) to form ent-progesterone.

One of ordinary skill in the art will recognize that there are many methods to convert olefins into vicinal diols. The present invention makes use of dihydroxylation reactions using osmium reagents including osmium tetroxide or manganese reagents, and reactions comprising a first step of forming an epoxide followed by a second step of hydrolyzing an epoxide. It will also be appreciated that there are many reagents useful for the oxidative cleavage of vicinal diols. Reagents useful for the oxidative cleavage of vicinal diols include, but are not limited to, sodium periodate and lead tetraacetate.

In an aspect not in accordance with the invention, there is provided a method for preparing ent-progesterone comprising the step of reacting a compound of formula A: 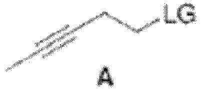 with metal bromide to produce 5-bromopent-2-yne, wherein LG represents a leaving group. In certain embodiments, the leaving group is selected from the group including but not limited to tosylate, mesylate, triflate, bromide, chloride and iodide. In certain other embodiments, LG is a tosylate group.

In an aspect not in accordance with the invention, there is provided a method for 5-bromopent-2-yne, the method comprising the step of reacting a compound of formula A: 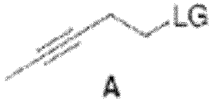 wherein LG represents a leaving group, with a metal bromide, to produce 5-bromopent-2-yne.

In certain embodiments of the above aspects, the leaving group is selected from the group including but not limited to tosylate, mesylate, triflate, bromide, chloride and iodide. In certain other embodiments, LG is a tosylate group.

In an aspect not in accordance with the invention, there is provided a method for preparing ent-progesterone comprising the step of reacting a compound of formula **D:** Et0-2( 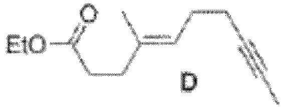 with such as diisobutylaluminum hydride to form a compound of formula **E:**

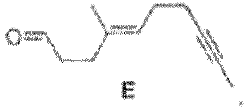

In an aspect not in accordance with the invention, there is provided a method for preparing a compound of formula **E:** 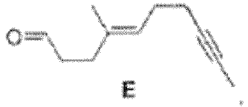 comprising the step of reacting a compound of formula **D:** 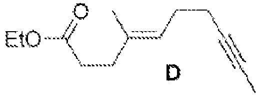 with a reducing agent such as diisobutylaluminum hydride to form the compound of formula **E.**

In an aspect not in accordance with the invention, there is provided a method for preparing ent- progesterone, the method comprising the step of reacting a compound of formula V: 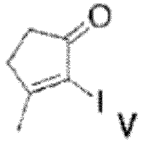 with propargyl alcohol to form a compound of formula **W:** 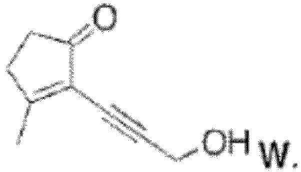 Such a reaction can be performed, e.g., under Sonogashira coupling conditions, e.g., in the presence of a palladium and/or copper catalyst.

In an aspect not in accordance with the invention, there is provided a method for preparing a compound of formula **W:** 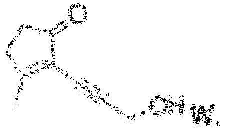 the method comprising the step of reacting a compound of formula **V:** 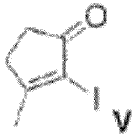 with propargyl alcohol to form the compound of formula **W.** As mentioned above, such a reaction can be performed, e.g., under Sonogashira coupling conditions, e.g., in the presence of a palladium and/or copper catalyst.

In an aspect not in accordance with the invention, there is provided a method for prepanng ent-progesterone comprising the step of hydrogenating a compound of formula **W:** 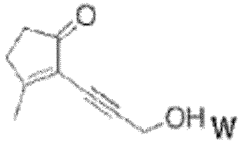 to form a compound of formula **X:**

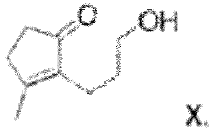

In an aspect not in accordance with the invention, there is provided a method for preparing a compound of formula X: 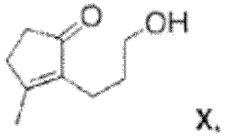 the method comprising the step of hydrogenating a compound of formula **W:** 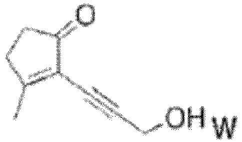 to form the compound of formula **X.**

In an aspect not in accordance with the invention, there is provided a method for preparing ent- progesterone comprising the step of reacting a compound of 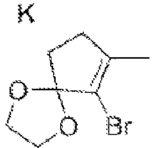 formula **K:**
with a compound of formula **M:** 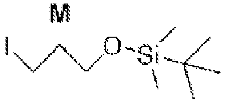 to form a compound of formula **N:**

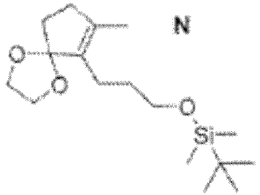

In an aspect not in accordance with the invention, there is provided a method for preparing a compound of formula **N:** 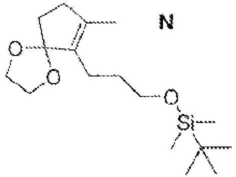 the method comprising the step of reacting a compound of formula **K:** 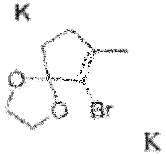 with a compound of formula **M:** 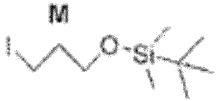 to form the compound of formula N.

In certain embodiments of the preceding aspects, the reaction of K and M is in the presence of a lithium compound. In other embodiments, the reaction of K and M is performed in the presence of a solvent. In certain other embodiments the solvent is dimethyl-2-imidazolidinone or 1,3-dimethy1-3,4,5,6-tetrahydro-2(lH)-pyrimidinone or hexamethylphosphoramide or mixtures thereof.

In an aspect not in accordance with the invention, there is provided a method for prepanng ent-progesterone comprising the step of reacting a compound of the formula U: 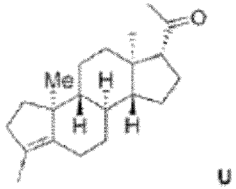 to form a triketone of formula **U'** 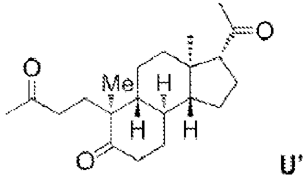 followed by cyclization of a triketone of formula U' (e.g., in the presence of base) to form ent-progesterone. The compound U can be prepared by a method comprising the step of reacting a compound of formula A: 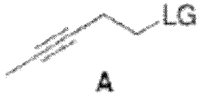 with metal bromide to produce 5-bromopent-2-yne, wherein LG represents a leaving group. In certain embodiments, the leaving group is selected from the group including but not limited to, tosylate, mesylate, triflate, bromide, chloride and iodide. In certain embodiments, LG is a tosylate group.

In an aspect not in accordance with the invention, there is provided a method for preparing ent-progesterone comprising the step of reacting a compound of the formula U: 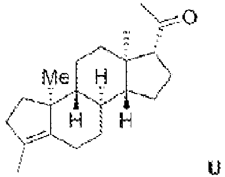 to form a triketone of formula **U'** 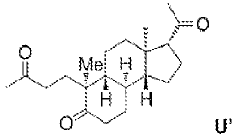 followed by cyclization of a triketone of formula **U'** (e.g., in the presence of base) to form ent-progesterone, wherein the compound U is prepared by a method comprising the step of reacting a compound of formula D: 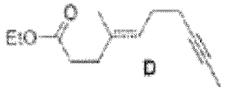 with diisobutylaluminum hydride to form a compound of formula E:

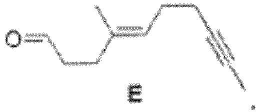

In an aspect not in accordance with the invention, there is provided a method for prepanng ent- progesterone comprising the step of reacting a compound of the formula U: 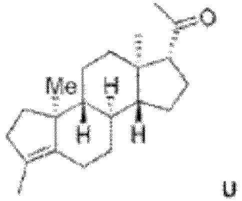 to form a triketone of formula **U'** 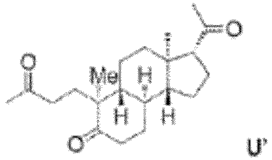 followed by cyclization of a triketone of formula **U'** (e.g., in the presence of base) to form ent-progesterone,
wherein the compound U is prepared by a method comprising the step of reacting a compound of formula **K: K** 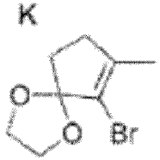 with a compound of formula M:

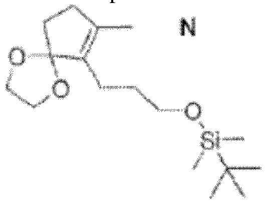

In an aspect not in accordance with the invention, there is provided a method for preparing ent- progesterone comprising the step of reacting a compound of the formula U: \,,,,-0 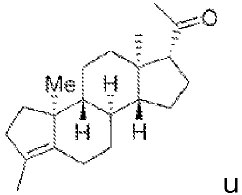 to form a triketone of formula **U'** 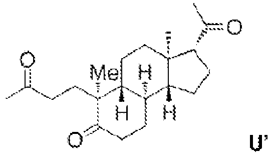 followed by cyclization of a triketone of formula **U'** (e.g., in the presence of base) to form ent-progesterone,
wherein the compound U is prepared by a method comprising the step of reacting a compound of formula H: 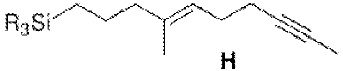 with a compound of formula S : 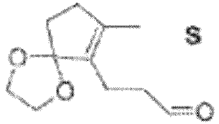 to form a compound of formula T: 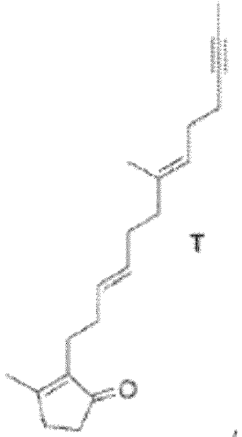 wherein each instance of R is independently a C1-C4 straight or branched alkyl group, or a C3-C8 cycloalkyl group. The compound of formula T can be used to prepare acompound of formula U, e.g., by addition of a methyl group synthon (using, e.g., methyl lithium or a methyl Grignard reagent such as methylmagnesium bromide) followed by cyclization (e.g., using an acid catalyst such as trifluoroacetic acid). In an aspect not in accordance with the invention, there is provided a method for preparing a compound of formula U: 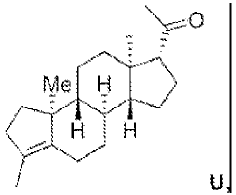 the method comprising the step of reacting a compound of formula H: 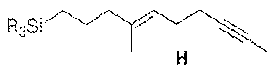 with a compound of formula S : 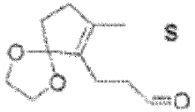 to form a compound of formula T: 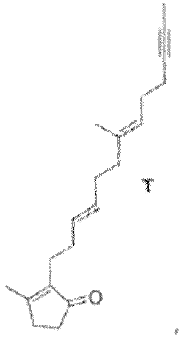 wherein each instance of R is independently a Cl-04 straight or branched alkyl group, or a C3-C8 cycloalkyl group, and cyclizing the compound of formula T to form the compound of formula U.

In an aspect not in accordance with the invention, there is provided a method for preparing a compound of formula T: 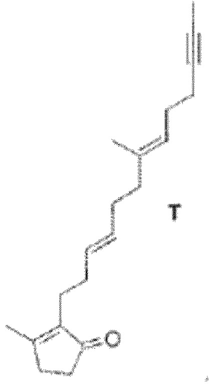 the method comprising the step of reacting a compound of formula H: 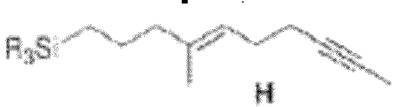 wherein each instance of R is independently a Cl-04 straight or branched alkyl group, or a C3-C8 cycloalkyl group, with a compound of formula S : 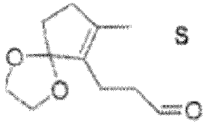 to form the compound of formula T.

In an aspect not in accordance with the invention, there is provided a method for preparing ent-progesterone comprising the step of reacting a compound of the formula U: 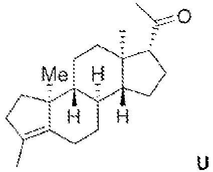 to form a triketone of formula **U'** 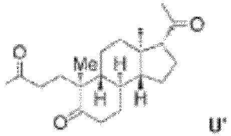 followed by cyclization of a triketone of formula U' to form ent-progesterone, wherein the compound U is prepared by a method comprising the step of reacting a compound of formula E: 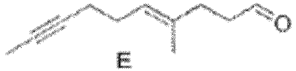 with a compound of formula R : 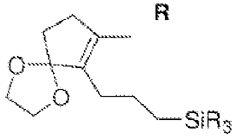 to form a compound of formula T: 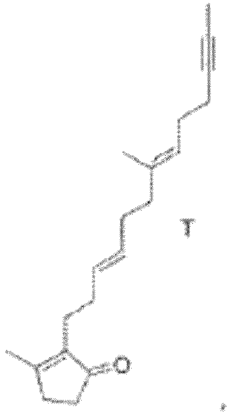 wherein each instance of R is independently a Cl-04 straight or branched alkyl group, or a C3-C8 cycloalkyl group.

In an aspect not in accordance with the invention, there is provided method for preparing a compound of formula T: 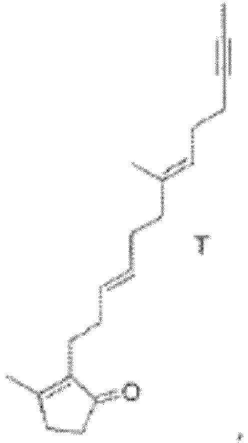 the method comprising the step of reacting a compound of the formula E: 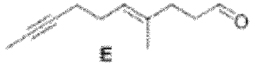 with a compound of formula R: 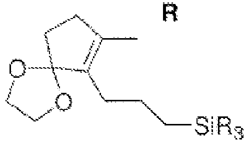 wherein each instance of R is independently a Cl-04 straight or branched alkyl group, or a C3-C8 cycloalkyl group,
to form the compound of formula **T.**

In an aspect not in accordance with the invention, there is provided a method for prepanng ent- progesterone comprising the step of reacting a compound of the formula U: 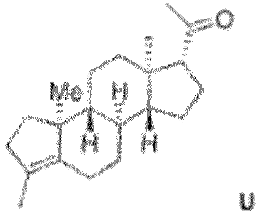 to form a triketone of formula **U'** 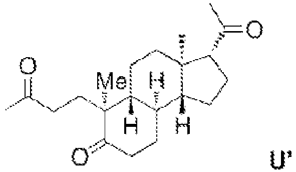 followed by cyclization of a triketone of formula U' to form ent-progesterone, wherein the compound U is prepared by a method comprising the step of reacting a compound of formula **V:** 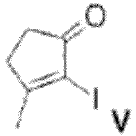 with propargyl alcohol to form a compound of formula **W:**

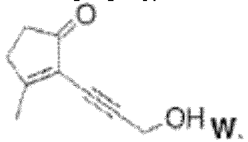

In an aspect not in accordance with the invention, there is provided a method for preparing a compound of formula **W:** 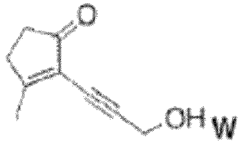 the method comprising the step of reacting a compound of formula **V:** 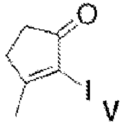 with propargyl alcohol to form the compound of formula **W**

In an aspect not in accordance with the invention, there is provided a method for prepanng ent- progesterone comprising the step of reacting a compound of the formula **U:** 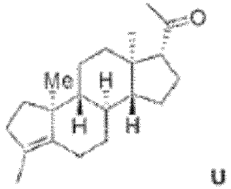 to form a triketone of formula **U'** 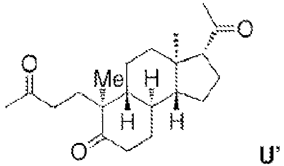 followed by cyclization of the triketone of formula **U'** (e.g., in the presence of base) to form ent-progesterone,
wherein the compound **U** is prepared by a method comprising the step of hydrogenating a compound of formula **W:** 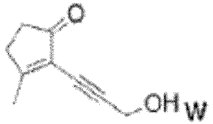 to form a compound of formula **X:**

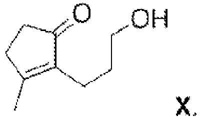

In an aspect not in accordance with the invention, there is provided a method for preparing a compound of formula **X:** 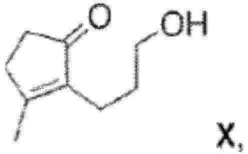 the method comprising the step of
hydrogenating a compound of formula **W:** 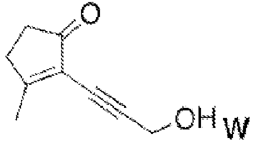 to form the compound of formula **X.**

In an aspect not in accordance with the invention, there is provided a method for prepanng ent- progesterone comprising the step of reacting a compound of the formula **U:** 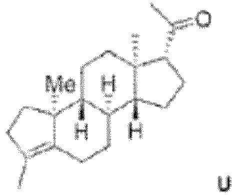 to form a triketone of formula **U'** 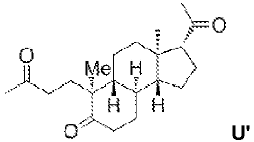 followed by cyclization of the triketone of formula **U'** (e.g., in the presence of base) to form ent-progesterone, wherein the compound U is prepared by a method comprising the step of reacting a compound of formula E: 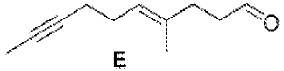 with a compound of formula Q : to form a compound of formula T:

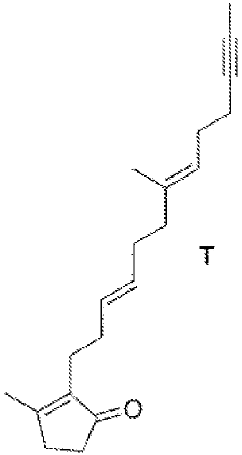

In an aspect not in accordance with the invention, there is provided a method for prepanng a compound of formula T: 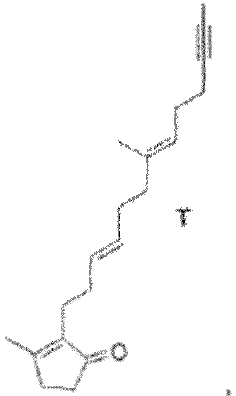 the method comprising the step of reacting a compound of formula E: 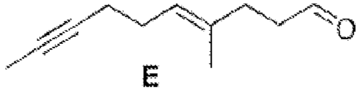 with a compound of formula Q : 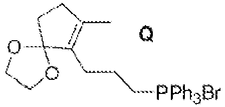 to form the compound of formula T.

In an aspect not in accordance with the invention, there is provided a method for preparing ent- progesterone comprising the step of reacting a compound of the formula U: 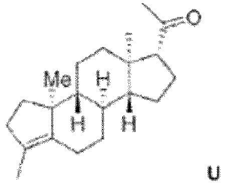 to form a triketone of formula **U'** 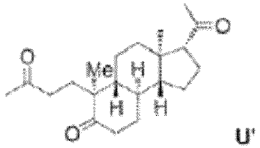 followed by cyclization of a triketone of formula **U'** (e.g., in the presence of base) to form ent-progesterone, wherein the compound U is prepared by a method comprising the step of reacting a compound of formula I: 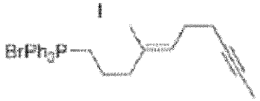 with a compound of formula S: 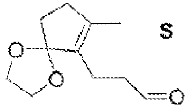 to form a compound of formula T:

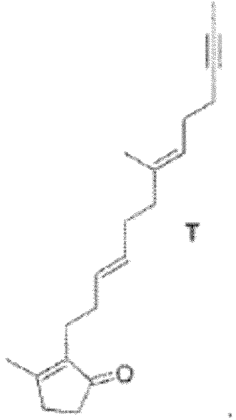

In an aspect not in accordance with the invention, there is provided a method for prepanng a compound of formula T: 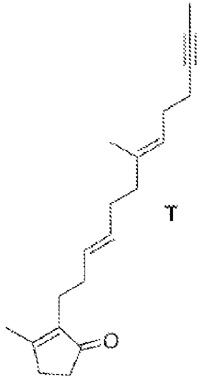 the method comprising the step of reacting a compound of formula I: 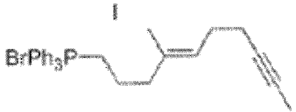 with a compound of formula S: 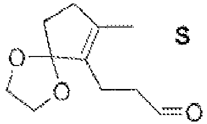 to form the compound of formula T.

In an aspect not in accordance with the invention, there is provided a method for preparing ent-progesterone comprising the step of reacting a compound of the formula **U:** 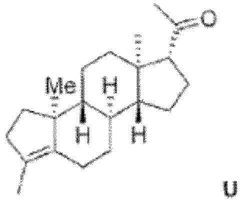 to form a triketone of formula **U'** 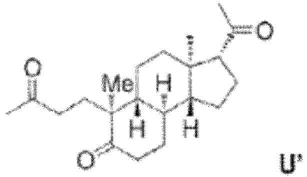 followed by cyclization of the triketone of formula **U'** (e.g., in the presence of base) to form ent-progesterone, wherein the compound U is prepared by a method comprising the step of reacting a compound of formula I: 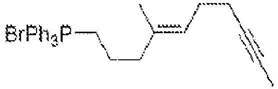 to form a compound of formula **Y:** 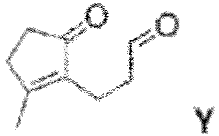 to form a compound of formula T:

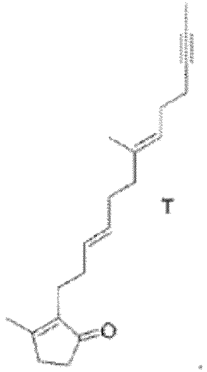

In an aspect not in accordance with the invention, there is provided a method for preparing a compound of formula T: 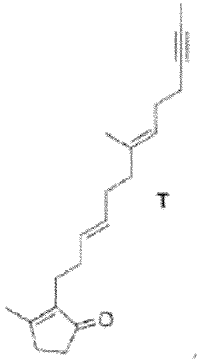 the method comprising the step of reacting a compound of formula I: 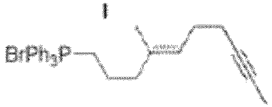 with a compound of formula **Y:** 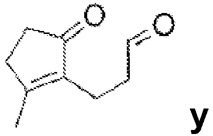 to form the compound of formula T. In certain embodiments, the step of reacting is performed in the presence of a strong base such as phenyllithium.

In accordance with other methods not in accordance with the invention, enantiomerically-enriched ent- progesterone may be obtained by separation of enantiomers, either of a racemic intermediate or of racemic progesterone. Thus, the present invention further contemplates a method of preparing ent-progesterone by isolating enantiomerically-enriched ent-progesterone from racemic progesterone, e.g., progesterone produced by any of the methods disclosed herein. The present invention also contemplates preparing ent-progesterone by reacting an enantiomerically-enriched intermediate, e.g., enantiomerically-enriched intermediate **U or U'** disclosed herein, and transforming the enantiomerically-enriched intermediate through one or more reaction steps to provide ent-progesterone.

In an aspect not in accordance with the invention, there is provided for one or more intermediates of the synthetic method of the invention. In certain aspects, the intermediate is a compound having one of the following formulas:

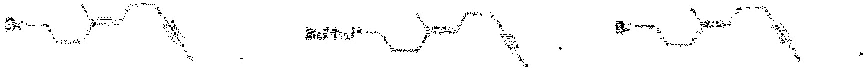

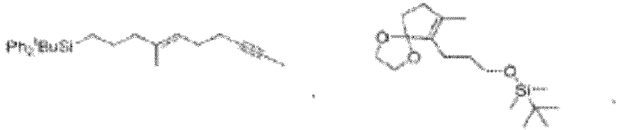

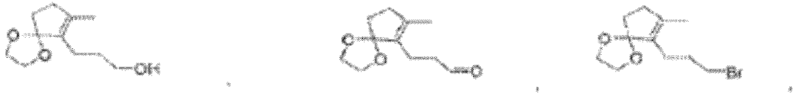

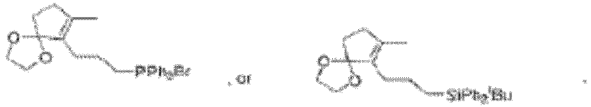

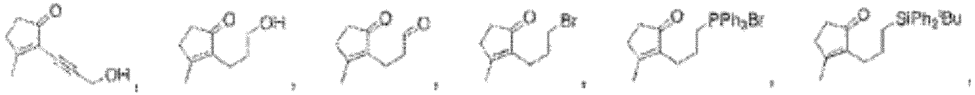

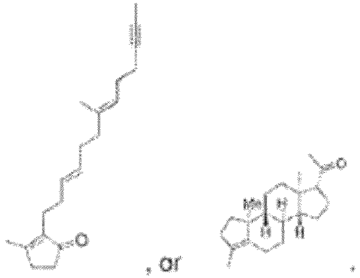

It should be further understood that the above summary of the present invention is not intended to describe each disclosed embodiment or every implementation of the present invention. The description further exemplifies illustrative embodiments. In several places throughout the specification, guidance is provided through examples, which examples may be used in various combinations. In each instance, the examples serve only as representative groups and should not be interpreted as exclusive examples.

### Detailed Description

By way of illustrating and providing a more complete appreciation of the present invention and many of the attendant advantages thereof, the following detailed description and examples are given concerning the novel syntheses for making ent-progesterone, individual novel steps within the syntheses and individual novel intermediates formed during the novel syntheses of the present invention.

As used in the description of the invention and the appended claims, the singular forms "a", "an" and "the" are used interchangeably and intended to include the plural forms as well and fall within each meaning, unless the context clearly indicates otherwise. Also, as used herein, "and/or" refers to and encompasses any and all possible combinations of one or more of the listed items, as well as the lack of combinations when interpreted in the alternative ("or").

As used herein, "at least one" is intended to mean "one or more" of the listed elements.

The term "alkyl" refers to a straight or branched hydrocarbon chain radical consisting solely of carbon and hydrogen atoms, containing no unsaturation, having from one to eight carbon atoms, and which is attached to the rest of the molecule by a single bond, such as illustratively, methyl, ethyl, n-propyl 1-methylethyl (isopropyl), n-butyl, n-pentyl, and 1,1-dimethylethyl (tert-butyl).

The term "cycloalkyl" denotes a non-aromatic mono or multicyclic ring system of 3 to 12 carbon atoms such as cyclopropyl, cyclobutyl, cyclopentyl, cyclohexyl and examples of multicyclic cycloalkyl groups include perhydronapththyl, adamantyl and norbornyl groups bridged cyclic group or spirobicyclic groups e.g. spiro(4,4)non-2-yl.

The term "leaving group," or "LG", as used herein, refers to any group that leaves in the course of a chemical reaction involving the group and includes but is not limited to halogen, brosylate, mesylate, tosylate, triflate, p-nitrobenzoate, phosphonate groups, for example.

Singular word forms are intended to include plural word forms and are likewise used herein interchangeably where appropriate and fall within each meaning, unless expressly stated otherwise.

Except where noted otherwise, capitalized and non-capitalized forms of a term fall within the meaning of the term.

Unless otherwise indicated, it is to be understood that all numbers expressing quantities, ratios, and numerical properties of ingredients, reaction conditions, and so forth used in the specification and claims are contemplated to be able to be modified in all instances by the term "about".

All parts, percentages, ratios, etc. herein are by weight unless indicated otherwise.

### General Preparative Methods

The particular process to be utilized in the preparation of the compounds used in this embodiment of the present invention depends upon the specific compound desired. Such factors as the selection of the specific substituents play a role in the path to be followed in the preparation of the specific compounds of this invention. Those factors are readily recognized by one of ordinary skill in the art.

The compounds of the present invention may be prepared by use of known chemical reactions and procedures. Nevertheless, the following general preparative methods are presented to aid the reader in synthesizing the compounds of the present invention, with more detailed particular examples being presented below in the experimental section describing exemplary working examples.

The compounds of the present invention may be made according to conventional chemical methods, and/or as disclosed below, from starting materials which are either commercially available or producible according to routine, conventional chemical methods. General methods for the preparation of the compounds are given below, and the preparation of representative compounds is specifically illustrated in examples.

Synthetic transformations that may be employed in the synthesis of certain compounds of this invention and in the synthesis of certain intermediates involved in the synthesis of compounds of this invention are known by or accessible to one skilled in the art. Collections of synthetic transformations may be found in compilations, such as:
J. March. Advanced Organic Chemistry, 4th ed.; John Wiley: New York (1992)
R.C. Larock. Comprehensive Organic Transformations, 2nd ed.; Wiley-VCH: New York (1999)
F.A. Carey; R.J. Sundberg. Advanced Organic Chemistry, 2nd ed.; Plenum Press: New York (1984)
T.W. Greene; P.G.M. Wuts. Protective Groups in Organic Synthesis, 3rd ed.; John Wiley: New York (1999)
L.S. Hegedus. Transition Metals in the Synthesis of Complex Organic Molecules, 2nd ed.; University Science Books: Mill Valley, CA (1994)
L.A. Paquette, Ed. The Encyclopedia of Reagents for Organic Synthesis; John Wiley: New York (1994)
A.R. Katritzky; 0. Meth-Cohn; C.W Rees, Eds. Comprehensive Organic Functional Group Transformations; Pergamon Press: Oxford, UK (1995)
G. Wilkinson; F.G A. Stone; E.W. Abel, Eds. Comprehensive Organometallic Chemistry; Pergamon Press: Oxford, UK (1982)
B.M. Trost; I. Fleming. Comprehensive Organic Synthesis; Pergamon Press: Oxford, UK (1991)
A.R. Katritzky; C.W. Rees Eds. Comprehensive Heterocylic Chemistry; Pergamon Press: Oxford, UK (1984)
A.R. Katritzky; C.W. Rees; E.F.V. Scriven, Eds. Comprehensive Heterocylic Chemistry 11; Pergamon Press: Oxford, UK (1996)
C. Hansch; P.G. Sammes; J.B. Taylor, Eds. Comprehensive Medicinal Chemistry: Pergamon Press: Oxford, UK (1990).

In addition, recurring reviews of synthetic methodology and related topics include Organic Reactions; John Wiley: New York; Organic Syntheses; John Wiley: New York; Reagents for Organic Synthesis: John Wiley: New York; The Total Synthesis of Natural Products; John Wiley: New York; The Organic Chemistry of Drug Synthesis; John Wiley: New York; Annual Reports in Organic Synthesis; Academic Press: San Diego CA; and Methoden der Organischen Chemie (Houben-Weyl); Thieme: Stuttgart, Germany. Furthermore, databases of synthetic transformations include Chemical Abstracts, each of which may be searched using either CAS OnLine or SciFinder, Handbuch der Organischen Chemie (Beilstein), and which may be searched using SpotFire, and REACCS.

The inventive methods of the present invention to make ent-progesterone are illustrated in Reaction Scheme 7. The reactions of the other Reaction Schemes are relevant as preferred embodiments, in the sense of how it is preferred to produce the starting materials of the process of the invention. The inventive methods include a number of intermediates and reaction methods which enable more efficient and less costly synthesis than heretofore known.

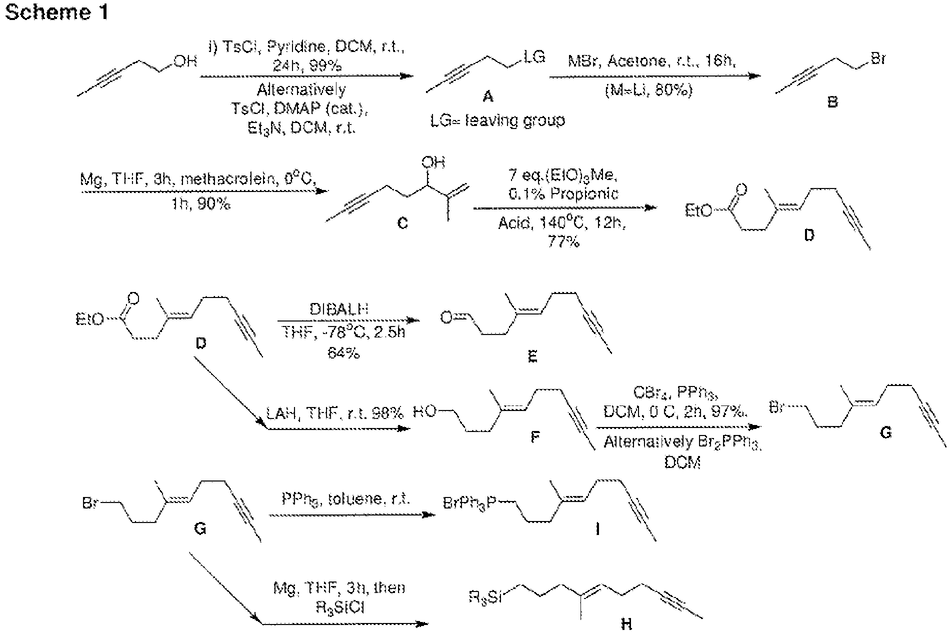

In Scheme 1, 3-pentyn-1-ol is converted to pent-3-ynyl 4-methylbenzenesulfonate (Intermediate A) by tosylation of the hydroxyl group. The tosyl group of Intermediate A is then brominated to form 5-bromopent-2-yne (Intermediate B). Intermediate B is reacted with methacrolein to produce 2-methyloct-1-en-6-yn-3-ol (Intermediate C) via a Grignard reaction. Intermediate C is reacted with trimethylorthoacetate to produce (E)-ethyl 4-methyldec-4-en-8-ynoate (Intermediate D).

Next, Intermediate D is reduced to form (E)-4-methyldec-4-en-8-ynal (Intermediate E) or (E)-4-methyldec-4-en-8-yn-1-ol (Intermediate F); either of which may be brominated to form (E)-10-bromo-7-methyldec-6-en-2-yne (Intermediate G).

Finally, Intermediate G is reacted to form an intermediate having a bulky phosphorous or silicon group: Intermediate I or Intermediate H.

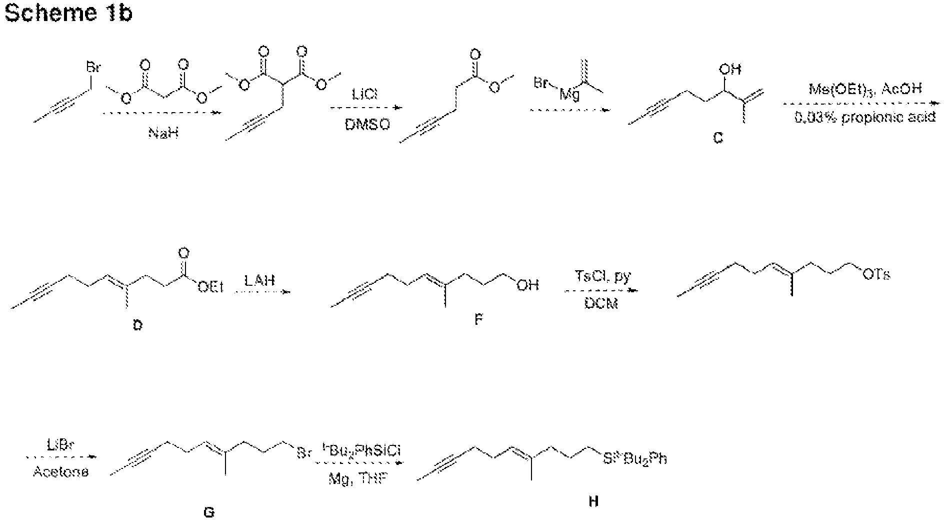

An alternative to Scheme 1 above, Intermediate H may be prepared as show in Scheme 1 b. In Scheme 1 b, Intermediate C is prepared by reacting 1-bromobut-2-yne with dimethylmalonate in the presence of sodium hydride to produce a substituted malonate which is then reacted with lithium chloride followed by a Grignard reagent.

Similarly, Intermediate G is prepared by tosylation followed by bromination of Intermediate F.

In the second phase of the synthesis, shown in Scheme 2 below, methyl cyclopentenone is converted to tert-butyldimethyl(3-(7-methyl-1,4-dioxaspiro[4.4]non-6-en-6-yl)propoxy)silane (intermediate N) via bromination of the double bond, followed by glycolization of the ketone.

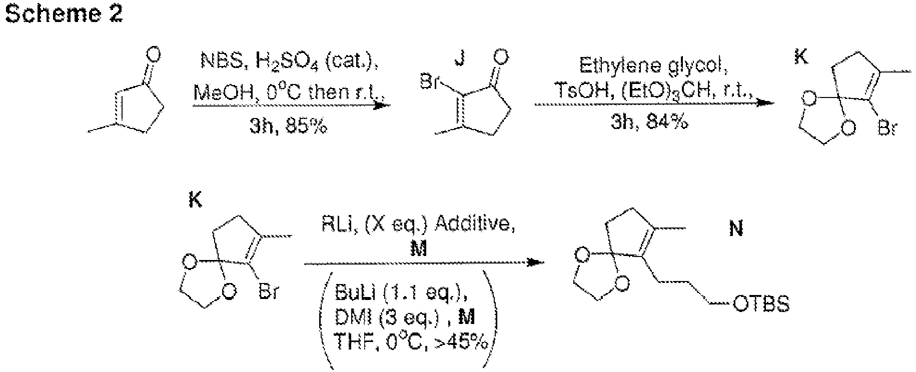

The conversion from 6-bromo-7-methyl-1,4-dioxaspiro[4.4]non-6-ene (Intermediate K) to intermediate N utilizes tert-butyl(3-iodopropoxy)dimethylsilane (Intermediate M) produced as shown in Scheme 3, below.

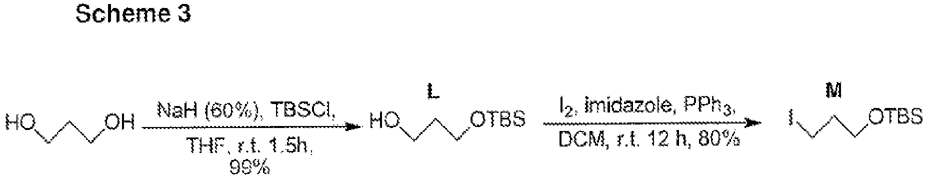

In Scheme 3, 1,3-propanediol is reacted with tert-butyldimethylsilyl chloride followed by reaction with Iodine to produce tert-butyl(3-iodopropoxy)dimethylsilane (Intermediate M). Compound N may also be prepared using an alternate preparation shown in Scheme 4, below.

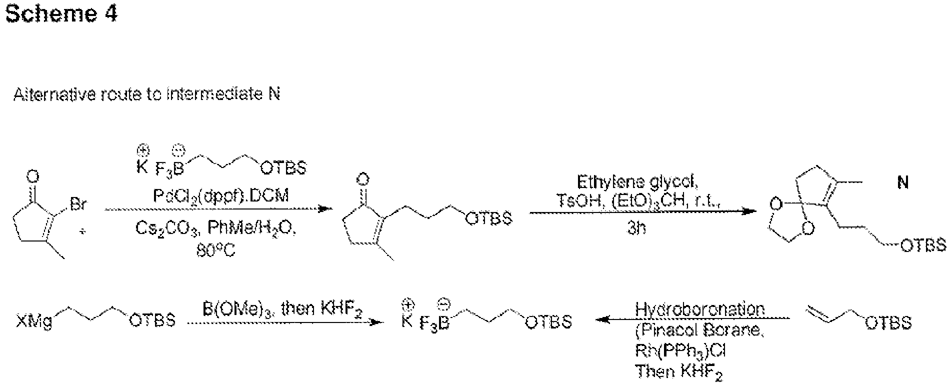

In Scheme 4, 2-bromo methyl cyclopentenone is converted to tea-butyldimethyl(3-(7-methyl-1,4-dioxaspiro[4.4]non-6-en-6-yl)propoxy)silane (Intermediate N) by converting the bromine group to the propylsilane group using a boron reagent (See, Molander, G. A.; Ham, J.; Seapy, D. G. Tetrahedron, 2007, 63, 768-775); which is followed by glycolization of the ketone.

In Scheme 5, shown below, intermediate N is converted to the hydroxyl intermediate (intermediate 0). Intermediate 0 is then converted to one of three intermediates: 3-(7-methyl-1,4-dioxaspiro[4.4]non-6-en-6-yl)propanal (intermediate S), or an intermediate having a bulky phosphorous or silicon group: Intermediate Q or Intermediate R; each of which may be utilized in the next phase of the reaction.

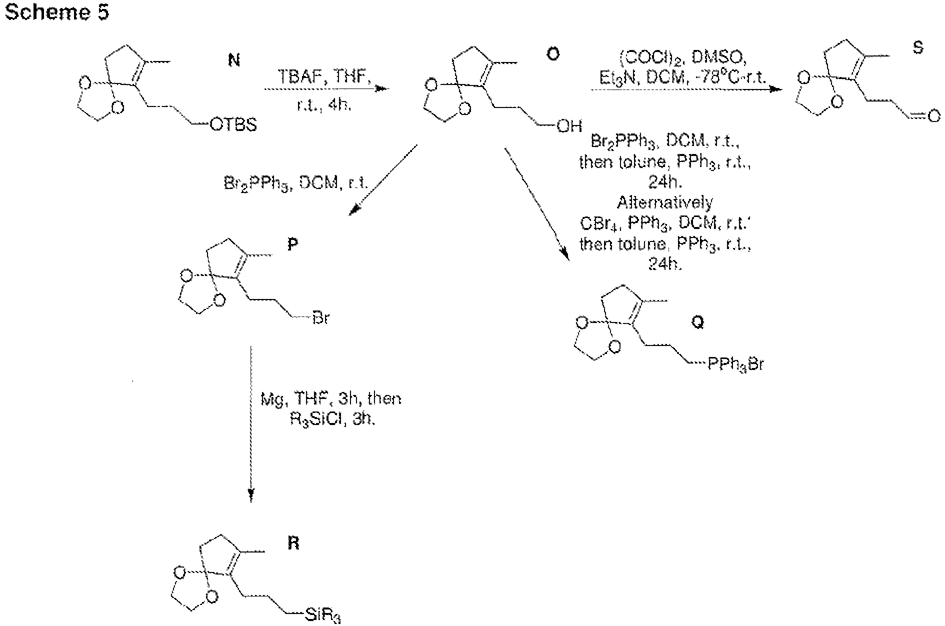

In scheme 6, shown below, 3-methyl-2-((3E,7E)-7-methyltrideca-3,7-dien-11-ynyl)cyclopent-2-enone (intermediate T) may be produced by one of two reaction approaches.

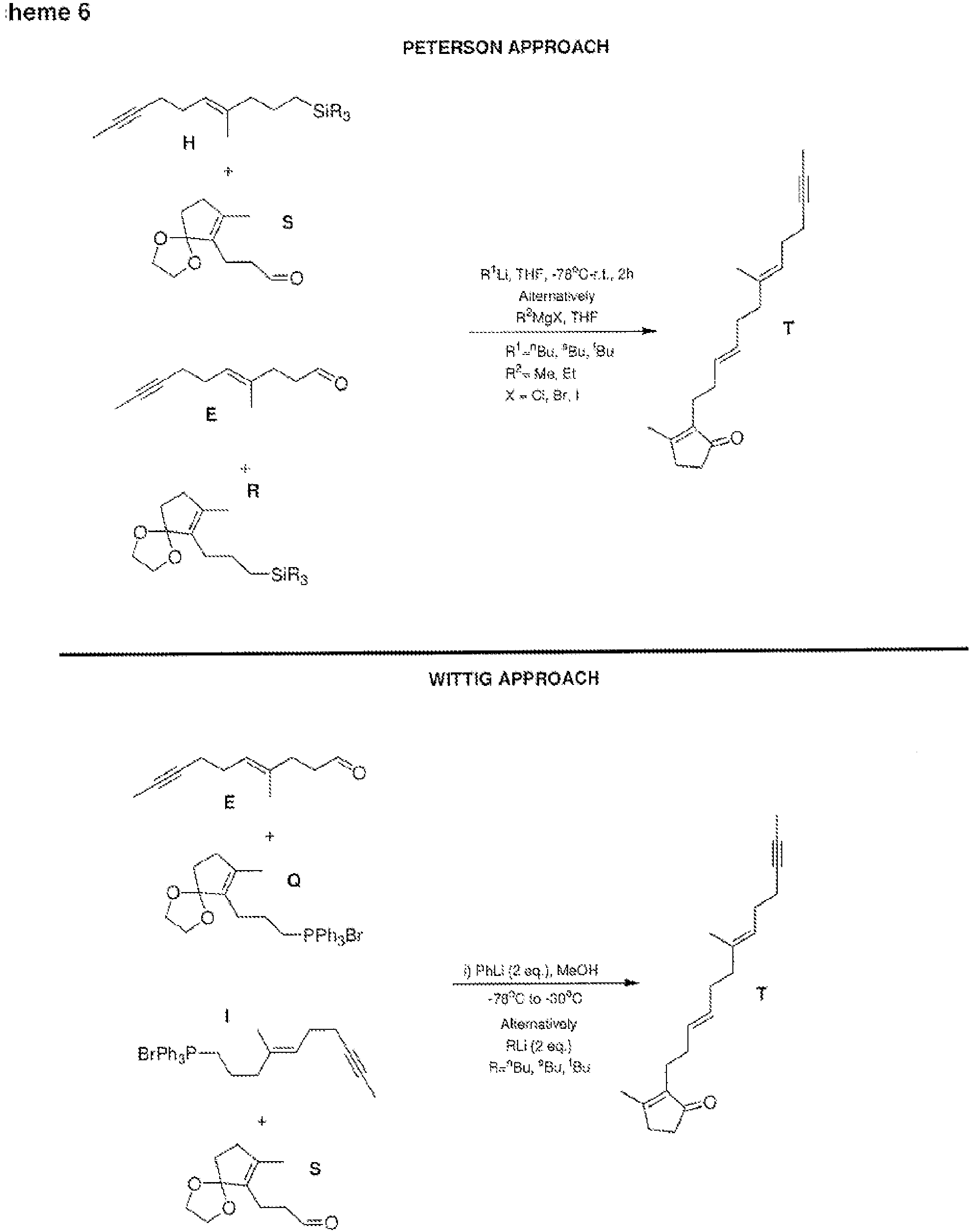

In the Peterson approach, W. Adam, C. M. Ortega-Schulte, Synlett, 2003, 414,416 and A. Barbero, Y. Blanco, C. Garcia, Synthesis, 2000, 1223-1228, Intermediates H and S or Intermediates E and R are reacted in the presence of s-butyl lithium to produce Intermediate T. This represents a new method in the synthesis of progesterones

In the Wittig approach, Johnson, W. S.; Gravestock, M. B.; McCarry, B. E. J. Am. Chem. Soc., 1971, 93, 4332, Intermediates E and Q or Intermediates I and S are reacted in the presence of phenyl lithium to produce Intermediate T.

In the final phase of the synthesis, shown in Scheme 7 shown below, Intermediate Tis cyclized to form a racemic mixture of l-((lR,3aR,3bR,8aS,8bR,10aR)-6,8a,10a-trimethyl-1,2,3,3a,3b,4,5,7,8,8a,8b,9,10,10a-tetradecahydrodicyclopenta[aJ]naphthalen-1-ypethanone and 1-((1S,3aS,3bS,8aR,8bS,10aS)-6,8a,10a-trimethyl-1,2,3,3a,3b,4,5,7,8,8a,8b,9,10,10a-tetradecahydrodicyclopenta[aJ]naphthalen-1-ypethan-1-one (Intermediate U, one enantiomer shown).

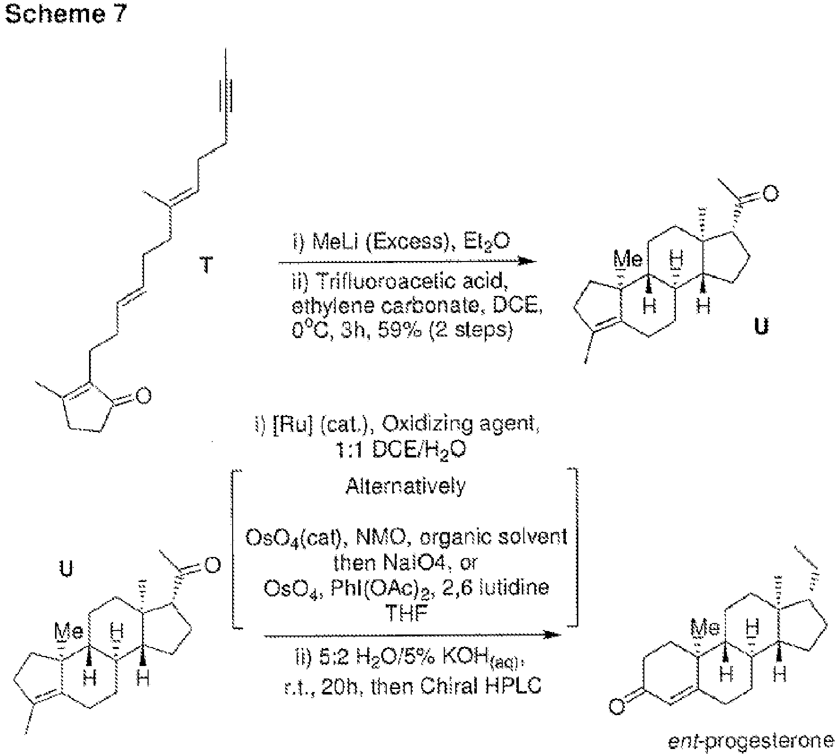

Intermediate U is then reacted to form a triketone intermediate of formula U': 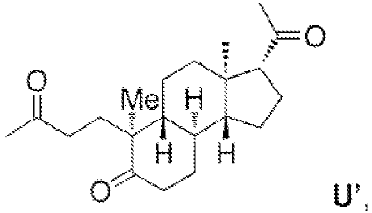 which is cyclized by treatment with a base, such as potassium hydroxide, to form ent-progesterone. It will be understood that the cyclization of T will produce racemic Compound U. The enantiomers of Compound U can be separated, if desired, and the appropriate enantiomer used in a further chiral synthesis of U' and ent-progesterone.

Thus, in one embodiment, the invention provides a method for preparing ent-progesterone, the method comprising the step of (a) reacting a compound of the formula U: 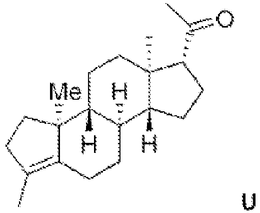 in which the compound of formula U is enantiomerically enriched, to form a triketone intermediate of the formula U', the process conditions being as defined in claim 1: 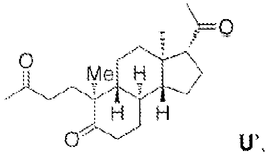 and (b) cyclizing the compound of formula U' to form ent-progesterone.

In another embodiment, the invention provides a method for preparing ent-progesterone, the method comprising the step of (a) reacting a compound of the formula U: 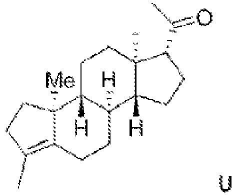 in which the compound of formula U is a racemic mixture with its enantiomer, to form a triketone intermediate of the formula U', the process conditions being as defined in claim 1: 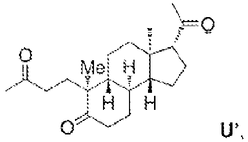 in which the compound of formula U' is a racemic mixture with its enantiomer,
(b) separating the enantiomers of the compound of formula U' to provide enantiomerically enriched compound of formula U'; and (c) cyclizing the enantiomerically enriched compound of formula **U'** to form ent-progesterone.

In yet another embodiment, the invention provides a method for preparing ent-progesterone, the method comprising the step of (a) reacting a compound of the formula U: 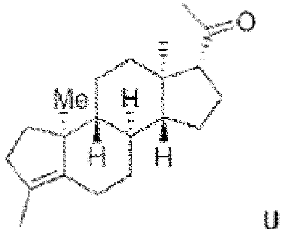 in which the compound of formula U 1s a racem1c mixture with its enantiomer, to form a triketone intermediate of the formula U', the process conditions being as defined in claim 1: 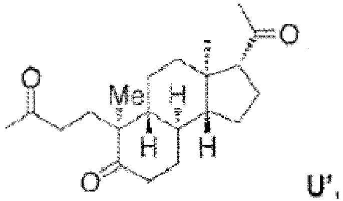 in which the compound of formula **U'** is a racemic mixture with its enantiomer,
(b) cyclizing the enantiomerically enriched compound of formula **U'** to form racem1c progesterone; and
(c) separating the enantiomers of racemic progesterone to form en-progesterone.

In certain embodiments not in accordance with the present invention, the conversion of **U** to **U'** is accomplished with a ruthenium catalyst and an oxidizing agent (such as sodium periodate). One of ordinary skill in the art will recognize that a useful ruthenium catalyst is ruthenium (III) chloride and that the ruthenium (III) chloride reaction can be performed in the presence of a solvent, including, but not limited to dichloroethane.

The conversion of **U** to **U'** is accomplished via a dihydroxylation reaction, using an osmium reagent or a manganese reagent, followed by the oxidative cleavage of a vicinal diol. In other embodiments, the conversion of **U** to **U'** is accomplished via a dihydroxylation reaction, using an osmium reagent or a manganese reagent, followed by the oxidative cleavage of a vicinal diol. Such methods relates to dihydroxylation reactions (e.g., using reagents such as osmium tetroxide (which may be used in catalytic amounts with a stoichiometric oxidant such as N-methylmorpholine N-oxide). It will also be appreciated that there are many reagents useful for the oxidative cleavage of vicinal diols. Reagents useful for the oxidative cleavage of vicinal diols include, but are not limited to, sodium periodate and lead tetraacetate.

Use of protecting groups can improve chemistry outcomes by eliminating the plurality of reactive sites in a given molecule. However, incorporation and subsequent removal of protecting groups adds synthetic steps. Synthetic routes that do not require protecting groups are therefore preferred. Scheme 8, shown below, illustrates the preparation of compounds **Y, AA** and **BB**, without the need for protecting groups.

As shown in Scheme 8, 3-methyl-2-cyclopentenone is iodinated on treatment with iodine and pyridinium dichromate to form compound **V.** One of ordinary skill in the art will recognize that there are multiple alternative methods for forming vinyl iodides. Compound **V** is subsequently coupled with propargyl alcohol utilizing Sonogashira conditions. The alkyne of the resulting ene-yne (compound **W**) is then selectively hydrogenated to form compound **X**. Finally, compound **X** is oxidized to the aldehyde compound **Y** under PCC conditions. One of ordinary skill in the art will recognize that there are multiple alternative methods for the oxidation of an alcohol to an aldehyde including, but not limited to, manganese dioxide, potassium permanganate, TEMPO, IBX and Swern.

Beginning with compound **X**, alternatives to compound **Y** include phosphonium salts and silanes. One of ordinary skill in the art will recognize that there are multiple methods useful for the conversion of alcohols to bromides or other halides. As illustrated in Scheme 8, compound **X** is converted to a bromide (compound **Z**) on treatment with carbon tetrabromide and triphenylphospine. On treatment with triphenylphospine, compound **Z** is converted to its corresponding triphenylphosphonium salt compound **AA**. Alternatively, compound **Z** is converted to a Grignard reagent on treatment with magnesium and the resulting Grignard reagent is reacted with tert-butyl diphenylsilyl chloride giving compound **BB**. Alternatives to Grignard reagents include, but are not limited to, organozinc reagents, organocuprates and alkyllithium reagents. It will be appreciated that organozinc reagents, organocuprates and alkyllithium reagents can all be prepared from alkyl bromides.

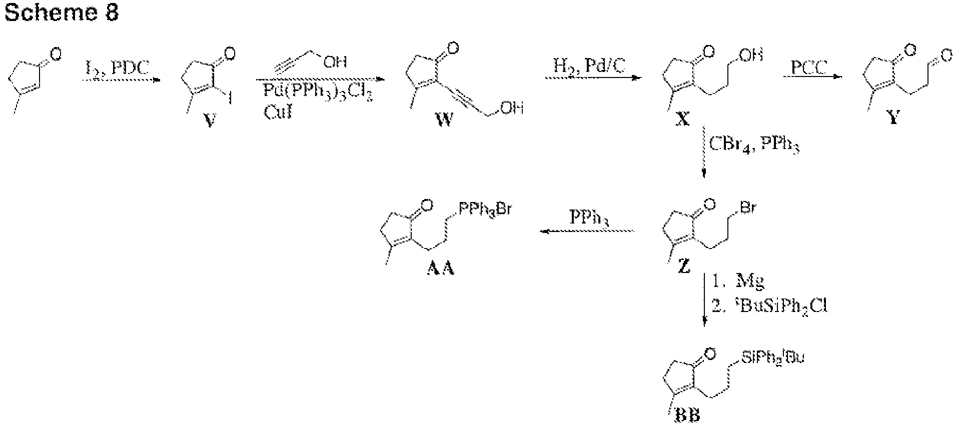

The aldehyde (compound **Y**), phosphonium salt (compound **AA**) and silane (compound BB) of Scheme 8 are useful for reactions analogous to those illustrated in Scheme 6 involving Wittig reactions and Peterson olefinations. A specific example is shown in Scheme 9, below. As illustrated, compound I (Scheme 1) is coupled with compound **Y** (Scheme 8) under Wittig conditions to generate compound T. Compound T, is then converted to ent-progesterone as illustrated in Scheme 7.

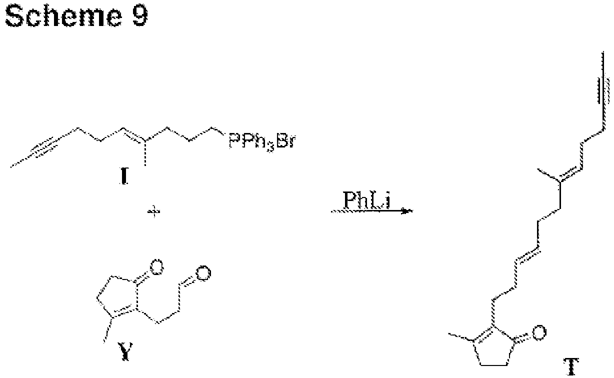

As described above, an enantiomerically-enriched ent-progesterone may be obtained by separation of enantiomers, either of a racemic intermediate (such as U or **U'**) or of racemic progesterone. Thus, the present invention further contemplates a method of preparing ent-progesterone by isolating ent-progesterone from racemic progesterone. The present invention also contemplates preparing ent-progesterone by reacting an enantiomerically-enriched intermediate, e.g., intermediate U or **U'** as disclosed herein, and transforming the enantiomerically-enriched intermediate through one or more reaction steps to provide ent-progesterone, provided that said process comprises the reaction steps defined in claim 1.

Separation of enantiomerically-enriched compounds, e.g., intermediates or progesterone, from a racemic mixture may be performed according to a variety of methods some of which are known in the art. For example, chiral high performance liquid chromatography (HPLC) and supercritical fluid chromatography (SFC) may be used to separate enantiomers. Chromatography columns having chiral stationary phases suitable for chiral HPLC or chiral SFC are commercially available. Alternatively, enantiomers may be separated by methods such as (i) recrystallization or complexation with a chiral material, followed by isolation of the enantiomer; (ii) derivatization with a chiral auxiliary and separation of diastereomers, followed by cleavage of the auxiliary and recovery of the enantiomer; (iii) resolution by selective reaction with an enantiomerically-enriched reagent, e.g., an enzyme or a chiral reduction of oxidation reagent, that modifies one enantiomer while leaving the other enantiomer substantially unchanged, followed by separation of the desired enantiomer.

Prior to the inventive method, the preparation of ent-progesterone from Intermediate U required the use of a dangerous and costly ozonolysis step. The inventive method of the present invention utilizes readily available materials and results in a compound having about >98% purity.

The present examples illustrate the invention as defined in the claims, but, in as far as the present examples relate to reaction steps involving the intermediates mentioned in claim 1, they use a different dihydroxylation agent to those specified in claim 1.

### Examples

### Abbreviations and Acronyms

A comprehensive list of the abbreviations used by organic chemists of ordinary skill in the art appears in The ACS Style Guide (third edition) or the Guidelines for Authors for the Journal of Organic Chemistry. For purposes of this invention, the chemical elements are identified in accordance with the Periodic Table of the Elements, CAS version, Handbook of Chemistry and Physics, 67th Ed., 1986-87.

More specifically, when the following abbreviations are used throughout this disclosure, they have the following meanings:
- atm: atmosphere
- br s: broad singlet
- Buchi: rotary evaporator ^{®}BUCHI Labortechnik AG
- C: Celsius
- CDCI3: deuterated trichloromethane
- Celite: diatomaceous earth filter agent ^{®}Celite Corp.
- d: doublet
- dd: doublet of doublets
- DIBAL-H: diisobutylaluminum hydride
- DCM: dichloromethane
- DMI: dimethyl-2-imidazolidinone
- g: gram
- h: hour, hours
- 1H NMR: proton nuclear magnetic resonance
- HPLC: high performance liquid chromatography
- J: coupling constant (NMR spectroscopy)
- L: liter
- LAH: lithium aluminum hydride
- 47:
- LG: leaving group
- M: mol L-1 (molar)
- m: multiplet
- MHz: megahertz
- min: minute, minutes
- mL: milliliter
- pM: micromolar
- mol: mole
- MS: mass spectrum, mass spectrometry

- m/z: mass-to-charge ratio
- N: equivalents L-1 (normal)
- NBS: N-bromo succinimide
- NMO: N-Methylmorpholine-N-Oxide
- NMR: Nuclear Magentic Resonance
- pH: negative logarithm of hydrogen ion concentration
- q: quartet
- RBF: round bottom flask
- rt: room temperature
- RT: retention time (HPLC)
- rt: room temperature
- s: singlet
- t: triplet
- THE: tetrahydrofuran
- TLC: thin layer chromatography
- TsCI: tosyl chloride

The percentage yields reported in the following examples are based on the starting components that are used in the lowest molar amount. Air and moisture sensitive liquids and solutions are transferred via syringe or cannula, and are introduced into reaction vessels through rubber septa. Commercial grade reagents and solvents are used without further purification. The term "concentrated under reduced pressure" refers to use of a Buchi rotary evaporator at 15 mm of Hg. All temperatures are reported uncorrected in degrees Celsius (°C). Thin layer chromatography (TLC) is performed on pre-coated glass-backed silica gel 60 A F-254 250 pm plates.

The structures of compounds of this invention are confirmed using one or more of the following procedures.

### NMR

NMR spectra are acquired for each compound when indicated in the procedures below. NMR spectra obtained were consistent with the structures shown.

Routine one-dimensional NMR spectroscopy was performed on either 300 or 500 MHz Varian^{®} Mercury-plus spectrometers. The samples were dissolved in deuterated solvents. Chemical shifts were recorded on the ppm scale and were referenced to the appropriate solvent signals, such as 2.49 ppm for DMSO-d6, 1.93 ppm for CD3CN, 3.30 ppm for CD3OD, 5.32 ppm for CD2Cl2 and 7.26 ppm for CDCI3 for 1H spectra.

### Materials

A VWR Dyastir magnetic stirrer is used for all reactions. Pyrex^{®} brand glassware is used unless otherwise stated. Chemicals and solvents that are used in the experimental workups are purchased from Sigma Aldrich, Fisher Scientific or EMD unless otherwise stated and the solvents used are either ACS or HPLC grade with the two grades being used interchangeably. For TLC analysis, the silica 60 gel glass backed TLC plates are purchased from EMD.

### Synthesis of Intermediate A

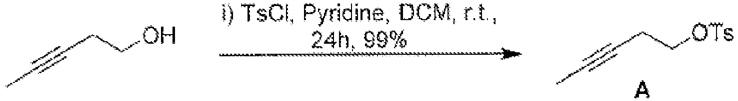

Compound A was prepared according to the method of Battenberg, O. A.; Nodwell, M. B.; Sieber, S. A. J. Org. Chem., 2011, 76, 6075-6087. To a dried, 1 L round bottom flask (RBF), equipped with a stirrer bar, under an atmosphere of Argon was added 250 mL of ACS grade dichloromethane (DCM) (Fisher Chemicals), 18.5 mL, 200 mmol, of 3-pentyn-1-ol (Sigma-Aldrich), 76 g, 400 mmol of tolunesulfonyl chloride (TsCI) and 45 mL of pyridine (Fisher Chemicals) sequentially. The reaction was then stirred for 18 h and was monitored by thin layer chromatography (TLC). After TLC analysis indicates the reaction has gone to completion the reaction mixture was quenched with 200 mL of a saturated, aqueous copper sulfate solution. The biphasic mixture was vigorously shaken and separated using a 1 L separatory funnel. The organic phase was collected and the aqueous phase was further extracted with two 75 mL portions of DCM. The combined organic phases are then washed with a sodium hydrogen carbonate (NaHCO3) and the aqueous layer was separated and extracted as before with two 75 mL portions of DCM. The combined organic phases are dried with sodium sulfate and filtered through a 250 mL sinter funnel into a 1 L mL RBF. The filtered residue was washed with a further 100 mL of DCM and the collected solution in the RBF was reduced under vacuum on a rotary evaporator (Buchi) to give Compound A as a clear oil. The proton nuclear magnetic resonance (NMR) spectrum in deuterated chloroform (CDCI3) matched the previously reported data. (See, Fang, F.; Vogel, M.; Hines, J. V.; Bergmeier, S. C.; Org. Biomol. Chem., 2012, 10, 3080-3091.)

### Synthesis of Intermediate B

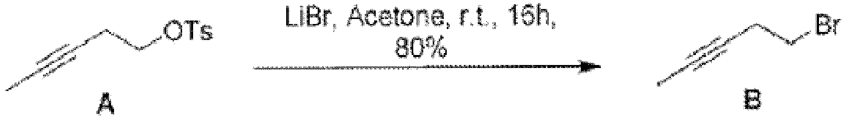

Compound **B** was prepared according to the method of Snider, B. B.; Kirk, T. C.; J. Am. Chem. Soc., 1983, 105, 2364-2368. To a dried 500 mL RBF, equipped with a stirrer bar, under an atmosphere of Argon was added 200 mL of ACS grade acetone (Fisher Chemicals) and 48 g, 200 mmol, of Compound **A.** The solution was stirred vigorously and cooled to 0°C with an ice bath whereupon 35 g of lithium bromide was added portion-wise over 5 minutes. The ice bath was removed after a further 10 minutes and the reaction allowed to warm to room temperature where it was stirred for a further 24 hours. After TLC analysis indicates the reaction has gone to completion the reaction mixture was diluted with 200 mL of hexane (EMI) and the mixture was filtered through a 250 mL sinter funnel with a 2.54cm (1 inch) plug of celite (Sigma-Aldrich) into a 500 mL RBF. The collected filtrate was then reduced under vacuum on a rotary evaporator (Buchi) to give Compound B as a clear oil. If a white precipitate was present the crude product was redissolved in hexane and the workup procedure repeated. The proton nuclear magnetic resonance (NMR) spectrum in CDCI3 matched the previously reported data. (See, Lubell, W. D.; Jamison, T. F.; Rapoport, H.J. Org. Chem., 1990, 55, 3511,3522.)

### Synthesis of Intermediate C

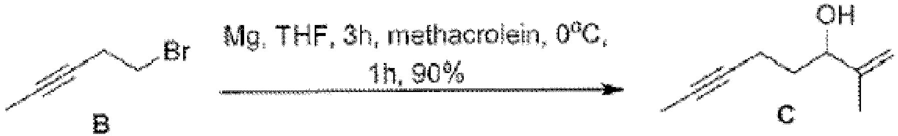

Compound **C** was prepared according to the method of Johnson, W. S.; Gravestock, M. B.; McCarry, B. E. J. Am. Chem. Soc., 1971, 93, 4332-4334. To a dried 500 mL RBF, equipped with a stirrer bar, under an atmosphere of Argon was added 120 mL of distilled THF, followed by 0.62 g, 31 mmol, of magnesium turnings (Sigma Aldrich) and the mixture was vigorously stirred at room temperature. Compound **B** (4.41g, 30 mmol) was the added to the flask via syringe in one portion and the reaction mixture was stirred at room temperature for 3 hours or until most of the magnesium has been consumed, whereupon the reaction mixture was cooled with an ice bath to 0°C. Meanwhile, in a separate, dried, 25 mL RBF 2.56 mL, 31 mmol, of methacrolein in 10 mL of distilled THF was cooled to 0°C with an ice bath. The methacrolein solution was then added to the Grignard solution via cannula over 10 minutes. The reaction mixture was then allowed to warm to room temperature and left for 1 hour. The reaction mixture was subsequently quenched with 75 mL of saturated, aqueous ammonium chloride solution and diluted with 150 mL of ethyl acetate. After being vigorously shaken, the biphasic mixture was then separated with a separatory funnel and the aqueous phase was further extracted with two 75 mL portions of ethyl acetate. The combined organic phases are then dried with sodium sulfate and filtered through a plug of 2.54cm (1 inch) of Celite and 2.54cm (1 inch) of flash silica (silica gel 60, EMD) via a 100 mL sinter funnel under vacuum into a 1 L RBF, with the sodium sulfate residue washed with a further 75 mL of ethyl acetate. The collected solution was then reduced under vacuum on a Buchi rotary evaporator to give compound C as a light yellow oil in 90% yield and >95% purity. The proton NMR spectrum in CDCI₃ agreed with the previously reported data. (See, Apparu, M.; Barrelle, M. Bulletin de la Societe Chimique de France, 1983, 3-4, Pt. 2, 83,86).

### Synthesis of Intermediate D

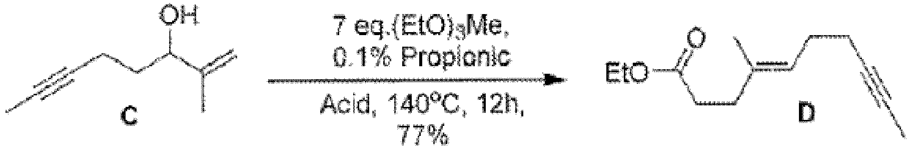

Compound **D** was prepared according to the method of Johnson, W. S.; Gravestock, M. B.; McCarry, B. E. J. Am. Chem. Soc., 1971, 93, 4332-4334. To a dried 150 mL RBF, equipped with a stirrer bar, under an atmosphere of Argon was added a solution of 4.14 g, 30 mmol, of Compound C in 7 molar equivalents of trimethylorthoacetate (Sigma-Aldrich) via syringe followed by 1 mol % of propionic acid (Sigma-Aldrich). The reaction vessel was fitted with a reflux condenser and the mixture was then heated to reflux with a 1200 mL Instatherm^{®} oil bath for 12 hours. The reaction was then removed from the oil bath and allowed to cool to room temperature. The crude product mixture was washed with a saturated sodium hydrogen carbonate solution (100 mL) and the aqueous layer removed via a 1 L separatory funnel, before being further extracted with 100 mL of ethyl acetate which was subsequently combined with the product mixture and reduced under vacuum on a Buchi rotary evaporator. Purification via short path distillation under reduced pressure gave Compound **D** as a clear oil in 71% yield and >95 purity. 1**H NMR (500 MHz, CDCI3): δ = 5.19 (tq, J = 6.8, 1.2, 1H), 4.10 (q, J = 7.2, 2H), 2.44-2.35 (m, 2H), 2.32-2.27 (m, 2H), 2.18-2.08 (m, 4H), 1.76 (t, J = 2.4, 3H), 1.61 (bs, 3H)**, **1.23 (t, J = 7.10, 3H).**

### Synthesis of Intermediate E

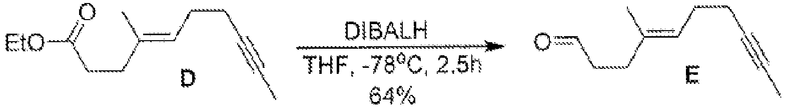

Compound **E** was prepared according to the method of Johnson, W. S.; Gravestock, M. B.; McCarry, B. E. J. Am. Chem. Soc., 1971, 93, 4332-4334. To a dried 150 mL RBF, equipped with a stirrer bar, under an atmosphere of Argon was added 50 mL of distilled THF and 2.08 g, 10 mmol, of Compound **D** and the mixture was cooled to -78°C in a dry-ice/acetone bath. After 15 minutes 12 mL, 12 mmol, of a 1 M solution of diisobutylaluminum hydride (DIBAL-H) in THF was added over 10 minutes and the reaction mixture was then left to stir for 2.5 hours. The reaction was then quenched with 5 mL of methanol at -78°C over 10 minutes and then allowed to warm to room temperature before 20 mL of water was added. The reaction mixture was extracted with 100 mL of ethyl acetate via a 1 L separatory funnel and the aqueous phase was further extracted with 2 more 50 mL portions of ethyl acetate and the combined extracts are dried with 100 g of sodium sulfate and reduced under vacuum on a Buchi rotary evaporator to give the crude product, Compound **E**, as a light yellow oil. Purification by flash column chromatography (Silica gel 60, EMD, 10:1 hexane/ethyl acetate) gave Compound E as a clear oil in 64% yield and >95% purity. **1H NMR (300 MHz, CDCI3): δ = 9.75 (t, J = 1.8, 1H), 5.20 (m, 1H), 2.52 (tm, J = 7.5, 2H), 2.32 (t, J = 7.5, 2H), 2.22¬2.07 (m, 4H), 1.76 (t, J = 2.4, 3H), 1.62 (bs, 3H**).

### Synthesis of Intermediate F

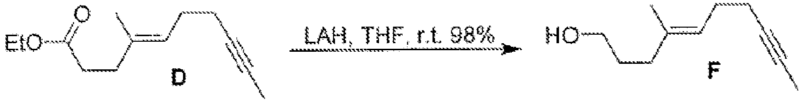

Compound **F** was prepared according to the method of Johnson, W. S.; Gravestock, M. B.; McCarry, B. E. J. Am. Chem. Soc., 1971, 93, 4332-4334. To a dried 1 L RBF, equipped with a stirrer bar, under an atmosphere of argon was added 250 mL of ether followed by 3.42 g, 90 mmol, of Lithium Aluminum Hydride (LAH, Sigma-Aldrich). The mixture was cooled to 0°C with an ice bath and after 15 minutes Compound **D** (9.0 g, 45 mmol), dissolved in 50 mL of ether was added over 10 minutes. After a further 1 hour or when TLC analysis indicates the reaction has gone to completion the reaction was quenched with 100 mL of 10% w/w aqueous sodium hydroxide solution over 5 minutes and then 50 mL of water before being brought to room temperature. The reaction mixture was extracted with 100 mL of ethyl acetate and the aqueous phase further with ethyl acetate (2 x 100 mL) utilizing a 1 L separatory funnel. The combined organic phases are dried with 50 g of sodium sulfate, filtered through a 100 mL sinter funnel, and reduced under vacuum on a rotary evaporator (Buchi) to give the crude product, Compound F, as a clear oil. Purification by flash column chromatography (Silica gel 60, EMD, 5:1 hexane/ethyl acetate) gave Compound **F** as a clear oil in 92% yield and >95% purity. **¹H NMR (500 MHz, CDCI3): δ= 5.22 (t, J = 6.8, 1H), 3.64 (t, J = 6.4, 2H), 2.21-2.11 (m, 4H), 2.08 (t, J = 7.5, 2H), t77 (bs, 3H), 1.68 (tt, J = 6.9, 6.9, 2H), 1.63 (s, 3H).**

### Synthesis of Intermediate G

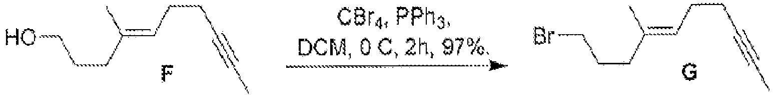

Compound **G** was prepared according to the method of Baughman, T. W.; Sworen, J. C.; Wagener, K. B. Tetrahedron, 2004, 60, 10943-10948. To a dried RBF, equipped with a stirrer bar, under an atmosphere of Argon was added 35 mL of DCM followed by 3.88 g carbon tetrabromide (Sigma-Aldrich) and 2.56 g of triphenyl phosphine (Sigma-Aldrich). The reaction mixture was cooled to 0°C with an ice bath and after 15 minutes 1.06 g of Compound **F**, dissolved in 10 mL of DCM was added over 5 minutes. After a further 2 hours or when TLC analysis indicates the reaction has gone to completion the reaction was diluted with 100 mL of hexane and filtered through 2.54cm (1 inch) of Celite via a 100 mL sinter funnel into a 500 mL RBF. The solution was reduced under vacuum on a Buchi rotary evaporator to give Compound G as a clear oil. If a white precipitate was present the crude product was redissolved in hexane and filtered through a plug of 2.54cm (1 inch) of Celite above 2.54cm (1 inch) of flash silica (silica gel 60, EMD) and reduced under vacuum to give Compound Gas a clear oil in 97% yield and >95% purity. **¹H NMR (500 MHz, CDCI3): δ= 5.21 (t, *J* = 6.8, 1H), 3.38 (t, *J* = 6.8, 2H), 2.20-2.11 (m, 6H), t98-t90 (m, 2H), t77 (bs, 3H), 1.61 (s**, **3H).**

### Synthesis of Intermediate H

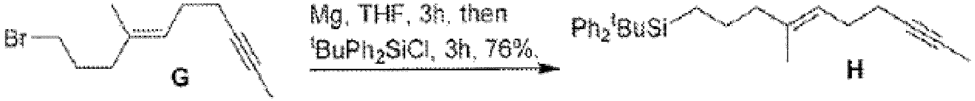

Compound **H** was prepared according to the method of Dixon, T. A.; Steele, K. P.; Weber, W. P. J. Organomet. Chem. 1982, 231, 299-305. To a dried 100 mL RBF, equipped with a stirrer bar, under an atmosphere of Argon was added 20 mL of distilled THF, followed by 50 mg of magnesium turnings (Sigma-Aldrich) and the mixture was vigorously stirred at room temperature. Compound **G** (0.46 g, 2 mmol) in 5 mL of distilled THF was the added to the flask via syringe in one portion and the reaction mixture was stirred at room temperature for 3 hours or until most of the magnesium has been consumed, whereupon 0.5 mL, 2 mmol, of *tert-*butyldiphenylsilyl chloride, dissolved in 5 mL of distilled THF, was added in one portion via syringe and the reaction was left to stir at room temperature for a further 3 hours. The reaction mixture was subsequently quenched with 50 mL of saturated, aqueous ammonium chloride solution and diluted with 100 mL of ethyl acetate and transferred to separatory funnel. After being vigorously shaken, the biphasic mixture was then separated and the aqueous phase was further extracted with two 50 mL portions of ethyl acetate. The combined organic phases are then dried with sodium sulfate and filtered through a plug of 2.54cm (1 inch) of Celite and 2.54cm (1 inch) of flash silica (silica gel 60, EMD) via a 100 mL sinter funnel under vacuum into a 500 mL RBF, with the sodium sulfate residue washed with a further 50 mL of ethyl acetate. The collected solution was then reduced under vacuum on a Buchi rotary evaporator to give compound **H**, crude, as a clear oil. Purification by flash column chromatography (Silica gel 60, EMD, hexane) gave Compound H as a clear oil in 76% yield and > 95% purity. **1H NMR (300 MHz, CDCI3): δ = 7.79-7.73 (m, 4H), 7.49-7.36 (m, 6H), 5.16 (t, J = 63, 1H), 2.24-2.09 (m, 4H), 1.96 (t, J = 7.5, 2H), 1.78 (t, J = 2.4, 3H), 1.60 (bs, 3H), 1.48-1.33 (m, 2H), 1.14 (s, 9H), 0.87 (t, J = 7.2, 2H).**

### Synthesis of Intermediate I

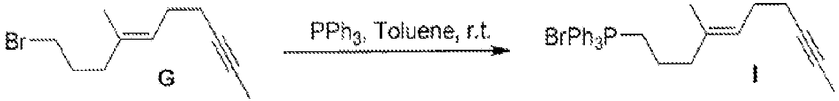

To a dried round bottom flask, equipped with a stir bar, under an atmosphere of Argon was added 0.228 g of Compound **G** in 5 mL of acetonitrile. To this solution was added 0.262 g of triphenylphosphine. A reflux condenser was attached and the reaction was heated to reflux under argon for 48 h. After cooling to room temperature the solvent was removed in vacuo. Benzene (30 mL) was added and the reaction was cooled to -20°C for 30 mins. After this time the product was filtered off as a white solid in 57% yield and >90% purity. **¹H NMR (300 MHz, CDCI3): δ = 7.90-7.60 (m, 15H), 5.21-5.10 (m, 1H), 3.80-3.65 (m, 2H), 2.29 (t, *J* = 6.6, 2H), 2.17-2.02 (m, 4H), 1.87 (s, 3H), 1.65-1.80 (m, 2H), 1.60 (s, 3H).**

### Synthesis of Intermediate J

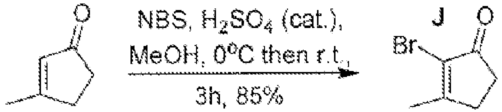

Compound **J** was prepared according to the method of Bliese, M.; Cristiano, D.; Tsanaktsidis, J. Aust. J. Chem., 1997, 50, 1043-1045. To a dried 1 L RBF, equipped with a stirrer bar, under an atmosphere of Argon was added 60 mL of methanol (Aldrich, HPLC grade) followed by 0.99 mL, 10 mmol, of 3-methyl cyclopentenone and 1.762 g, 9.9 mmol, of N-bromo succinimide. The reaction mixture was cooled to 0°C with an ice bath over 15 minutes, whereupon conc. sulfuric acid (0.2 eq.) was added and the reaction was left to stir for 3 hours being allowed to warm to room temperature over this time. Subsequently 50 mL of saturated sodium hydrogen carbonate and 40 mL of DCM are added and the mixture was transferred to a separatory funnel. After being vigorously shaken, the biphasic mixture was then separated and the aqueous phase was further extracted with two 50 mL portions of DCM. The combined organic phases are then dried with sodium sulfate and filtered through a plug of 2.54cm (1 inch) of Celite and 2.54cm (1 inch) of flash silica (silica gel 60, EMD) via a 100 mL sinter funnel under vacuum into a 500 mL RBF, with the sodium sulfate residue washed with a further 50 mL of DCM. The collected solution was then reduced under vacuum on a Buchi rotary evaporator to give compound J, crude, as a light yellow solid. Purification by flash column chromatography (Silica gel 60, EMD, hexane) gave Compound J as a cream crystalline in 85% yield and >98% purity. The **¹H** NMR spectrum in CDCI3 agreed with the previously reported data. (see, Bliese, M.; Cristiano, D.; Tsanaktsidis, J. Aust. J. Chem., 1997, 50, 1043-1045.)

### Synthesis of Intermediate K

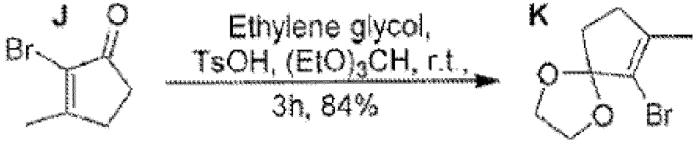

Compound **K** was prepared according to the method of Richter, A.; Hedberg, C.; Waldmann, H.J. Org. Chem., 2011, 76, 6694-6702. To a 500 mL rbf, equipped with a stirrer bar, under an atmosphere of Argon was added 200 mL of triethyl orthoacetate (Aldrich), 7.8 g, 40 mmol, of Compound **J** and 38 mg, 0.2 mmol of para-toluenesulfonic acid. The reaction mixture was stirred at room temperature for 3 hours or until TLC analysis indicates the reaction has gone to completion and the product was distilled off under vacuum to give Compound **K** as a clear oil which solidified to a white solid on cooling in 88% yield >96% purity. The ¹H NMR spectrum in CDCI₃ agreed with the previously reported data. (See, Richter, A.; Hedberg, C.; Waldmann, H. J. Org. Chem., 2011, 76,6694-6702.)

### Synthesis of Intermediate L

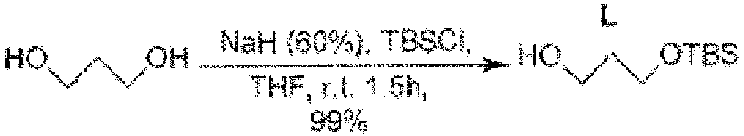

Compound L was prepared according to the procedure of McDougal, P. G.; Rico, J. G.; Oh, Y.-1.; Condon, B. J. Org. Chem., 1986, 51, 3388-3390. To a dried 250 mL RBF under an atmosphere of argon at room temperature was added 100 mL of distilled THF and 2.1 g of sodium hydride (60% dispersion in mineral oil; Aldrich). The mixture was stirred vigorously and 1,3-propanediol (4.0 g, 50 mmol; Aldrich) was added over 10 minutes via syringe. The reaction was allowed to stir for 45 minutes before tert-butyldimethylsilyl chloride (7.9 g, 52.7 mmol; Aldrich) was added portion wise over 5 minutes. The reaction was then allowed to stir for a further 45 minutes at room temperature before being quenched slowly with 20 mL of 10% aqueous sodium carbonate solution. This mixture was then transferred to a separatory funnel. After being vigorously shaken, the biphasic mixture was separated and the aqueous phase was further extracted with two 50 mL portions of ether. The combined organic phases are then dried with sodium sulfate and filtered through a plug of 2.54cm (1 inch) of Celite and 2.54cm (1 inch) of flash silica (silica gel 60, EMD) via a 100 mL sinter funnel under vacuum into a 500 mL RBF, with the sodium sulfate residue washed with a further 50 mL of ether. The collected solution was then reduced under vacuum on a Buchi rotary evaporator to give compound L, as a light yellow oil in 99% yield and >95% purity. The ¹H NMR spectrum in CDCI3 agreed with the previously reported data. (See, McDougal, P. G.; Rico, J. G.; Oh, Y.-1.; Condon, B. J. Org. Chem., 1986, 51, 3388-3390.)

### Synthesis of Intermediate M

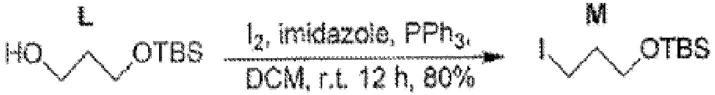

Compound **M** was prepared according to the procedure of Jakobsche, C. E.; Peris, G.; Miller, S. J. Angew. Chemie., Int. Ed., 2008, 47, 6707. To a dried 100 mL RBF under an atmosphere of argon at room temperature was added 25 mL of HPLC grade DCM, 0.81 g (5 mmol) of Compound **L,** 0.37 g (5.5 mmol) of imidazole (Aldrich), 1.45 g (5.5 mmol) of triphenylphosphine (Aldrich) and 1.4 g (5.5 mmol) of iodine (Fisher Chemicals). The reaction mixture was then stirred at room temperature for 12 hours, after which time it was diluted with hexane (100 mL) and filtered through a plug of 2.54cm (1 inch) of Celite and 5.08cm (2 inches) of flash silica (silica gel 60, EMD) via a 100 mL sinter funnel under vacuum into a 500 mL RBF. The collected solution was then reduced under vacuum on a Buchi rotary evaporator to give compound **M,** as a light clear oil in 80% yield and >95% purity. Residual triphenylphosphine may be removed by re-dissolving the product in hexane and filtering through another Celite/silica plug as described above. The ¹H NMR spectrum in CDCIJ agreed with the previously reported data. (See, Jakobsche, C. E.; Peris, G.; Miller, S. J. Angew. Chemie., Int. Ed., 2008, 47, 6707.)

### Synthesis of Intern1ediate N

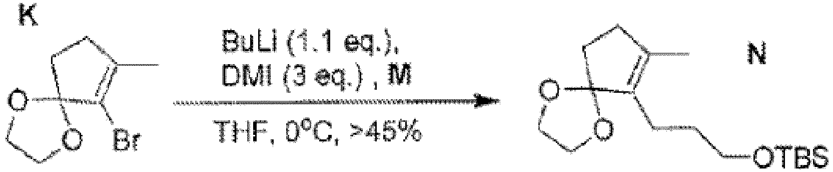

Compound **N** was prepared according to the procedure of Smith Ill, A. B.; Branca, S. J.; Pilla, N. N.; Guaciaro, M. A. J. Org. Chem., 1982, 47, 1855-1869, adapted with HMPA substituted for DMI. (see: Lo, C.-C.; Chao, P.-M. J. Chem. Ecology., 1990, 16, 3245-3253.) To a dried 100 mL RBF, equipped with a stirrer bar, under an atmosphere of argon was added 25 mL of distilled THF which was then cooled to -78°C with a dry ice bath. Then 3.44 mL, 5.5 mmol, of 1.6 M solution n-Butyllithium in hexanes (Aldrich) was added via syringe and the solution was allowed to stir for a further 15 minutes. Compound **K** (1.1 g, 5 mmol) was then added in 5 mL of distilled THF over 5 minutes and the reaction was allowed to stir at -78°C for a further 1 hour. After this time 3 equivalents of 1,3-Dimethyl-2-imidazolidinone (DMI), (1.71 mL, 15 mmol), was added drop-wise to the reaction mixture followed by, 30 minutes later, 1.36 g, 5 mmol, of Compound **M** dissolved in 5 mL of THF which was added over 10 minutes. The reaction was then left to stir until TLC analysis indicates complete consumption of the starting material during which time it was allowed to warm to -55°C and subsequently quenched with 25 mL of a saturated aqueous sodium dihydrogen phosphate solution. The reaction mixture then warmed to room temperature and diluted with 75 mL of ether and the mixture was transferred to a separatory funnel. After being vigorously shaken, the biphasic mixture was then separated and the aqueous phase was further extracted with two 50 mL portions of ether. The combined organic phases are then dried with sodium sulfate and filtered through a plug of 2.54cm (1 inch) of Celite and 2.54cm (1 inch) of flash silica (silica gel 60, EMD) via a 100 mL sinter funnel under vacuum into a 500 mL RBF, with the sodium sulfate residue washed with a further 50 mL of ethyl acetate. The collected solution was then reduced under vacuum on a Buchi rotary evaporator to give compound **N,** crude, as a light yellow oil. Purification by flash column chromatography (Silica gel 60, EMD, 5:1 hexane/ethyl acetate) gave Compound **N** as a light yellow oil in 45% yield and > 98% purity by GC-MS. GC-MS: 10.57 min, m[H]⁺ = 313.2.

### Combined Synthesis of Intermediate N

An alternative synthesis of compound **N,** which may be referred to as a "combined synthesis," is shown below in Scheme 10. 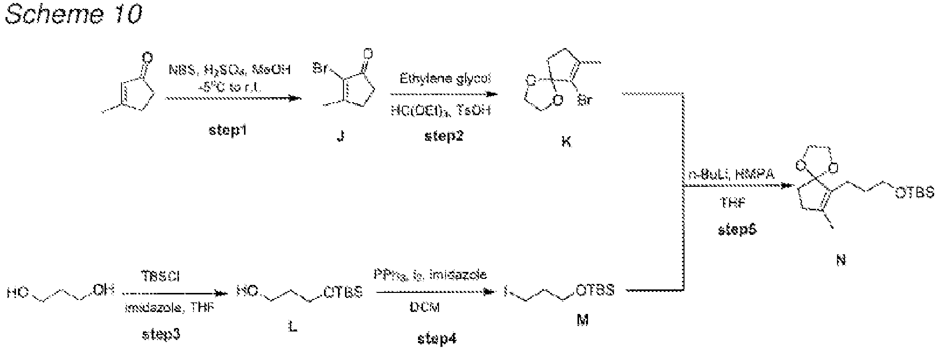 A detailed description of each of the five steps of Scheme 10 is provided below.

### (Step 1)

Under nitrogen atmosphere, charge 3-Methyl-2-cyclopenten-1-one (1.0 eq) and Me0H (6.0 v) to the reactor with stirring. Batchwise charge NBS (0.99 eq) at 1525 °C, then charge con.H2SO4 (0.02eq) below 5 °C. Stir the system at 15-25°C until the reaction completed shown on TLC. Charge sat.NaHCO3 (6.0 v) and DCM (4.0 v) to the system and stir for 10 mins. Separate and extract the water layer with DCM (2.0 v) twice. Combine the organic layer and wash with brine (6.0 v). Separate and collect the organic layer. Charge concentrated HCI (2.5 v) to the organic layer and stir for 20 hrs at r.t., separate and extract the water layer with DCM (2.0 v) twice. Combine the organic layer and wash with brine (6.0 v). Dry the organic layer with Na2SO4. Filter and concentrate the filtrate under vacuum at 3035 °C. The residue was recrystallized in PE/EA=0.8 v/1.2 v to give solid product of Intermediate J. The yield was 85%.

### (Step 2)

Charge Intermediate J (1.0 eq), triethyl orthoformate (3.5 eq), glycol (7.0 eq) and TsOH (0.01 eq) to reactor under N2. Stir at 20-25°C for 16 hrs. Charge sat.NaHCO3 (5.0 v) and cyclohexane (4.0 v) to the system. Stir for 10 min and separate. Extract the water layer with cyclohexane (3.0 v) twice and combine the organic layer. Wash the organic layer with brine (4.0 v). Dry the organic layer with Na₂SO₄. Filter and concentrate the filtrate under vacuum. Distill the residue under 5 mmHg to get the product of Intermediate K. The yield was 88%.

### (Step 3)

Charge propanediol (4.0 eq), THF (8.0 v) and imidazole (1.0 eq) to reactor. Charge TBSCI (1.0 eq) dropwise at -2~2°C. stir at -2~2°C for 2 hrs and then 20~25°C for 3 hrs. Charge water (10.0 v) and EA (5.0 v) to system. Stir for 10 mins and separate. Extract the water layer with EA (2.0 v) twice and combine the organic layer. Wash the organic layer with brine (4.0 v) and dry with Na2SO4. Filter and concentrate the filtrate under vacuum to give the crude product of Intermediate L used directly for next step.

### (Step 4)

Charge crude Intermediate L (1.0 eq), DCM (10.0 v), imidazole (1.5 eq) and PPh3 (1.5 eq) to reactor. Charge 12 (1.5 eq) at 0-5 °C. stir at 0-5°C for 0.5 hrs then 2025 °C for 0.5 hrs. Charge water (5.0 v) to system and stir for 10 mins. Separate and wash the organic layer with brine (5.0 v) twice. Dry the organic layer with Na2SO4. Filter and concentrate the filtrate under vacuum. The residue was purified by column to give the oil product of Intermediate M. The yield for 2 steps was 80%.

### (Step 5)

Charge Intermediate K (1.0 eq) and THF (10.0 v) to reactor under N2. Cool the system below - 78 °C. Charge n-BuLi (1.5 eq) dropwise below -70 °C and stir for 1 h. Charge HMPA (3.0 eq) dropwise below -65 °C and stir for 0.5 hrs. Charge PH-PRV-1301-102 (1.0 eq) dropwise below - 65 °C and stir for 5 hrs at -60-50°C. Charge water (20.0 v) and EA (5.0 v). Stir for 10 mins and separate. Extract the water layer with EA (2.0 v) twice and combine the organic layer. Wash the organic layer with brine (5.0 v). Dry the organic layer with Na₂SO₄. Filter and concentrate the filtrate under vacuum to give the crude product of Intermediate M (crude yield ~= 96% and purity -.55%).

### Synthesis of Intermediate O

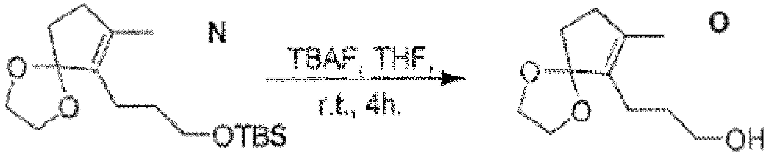

To a 500 mL RBF, equipped with a stirrer bar, under an atmosphere of argon at room temperature is added 150 mL of THF (ACS grade), 15.6 g, 50 mmol of Compound N and 100 mL of a 1 M solution of tetrabutylammonium fluoride (TBAF) in THF (Sigma-Aldrich). The reaction is stirred at room temperature for 4 hours or until TLC analysis indicates the reaction has gone to completion, whereupon 150 mL of water and 150 mL of ethyl acetate are sequentially added. This mixture is then transferred to a separatory funnel. After being vigorously shaken, the biphasic mixture is separated and the aqueous phase is further extracted with two 50 mL portions of ethyl acetate. The combined organic phases are then dried with sodium sulfate and filtered through a plug of 2.54cm (1 inch) of Celite via a 100 mL sinter funnel under vacuum into a 500 mL RBF, with the sodium sulfate residue washed with a further 50 mL of ethyl acetate. The collected solution is then reduced under vacuum on a Buchi rotary evaporator to give compound **O.**

### Synthesis of Intermediate P

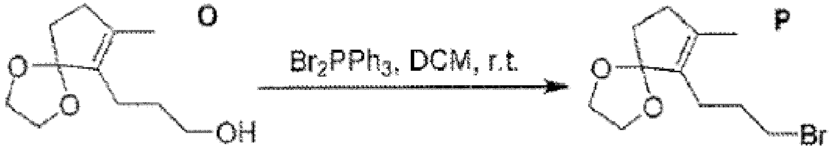

Compound **P** may be prepared by adapting the procedures of Lubell, W. D.; Jamison, T. F.; Rapoport, H.J. Org. Chem., 1990, 55, 3511-3522. To a 500 mL RBF, equipped with a stirrer bar, under an atmosphere of argon at room temperature is added 200 mL of distilled DCM, 9.9 g, 50 mmol of Compound **O** and 42.2 g, 100 mmol, of dibromotriphenylphosphorane (Sigma-Aldrich). The reaction mixture is stirred at room temperature and monitored by TLC analysis. An ice bath may be added at the beginning to prevent an exotherm. Once TLC analysis indicates the reaction has gone to completion the reaction mixture is filtered through a plug of 2.54cm (1 inch) of Celite and 2.54cm (1 inch) of flash silica (silica gel 60, EMD) via a 100 mL sinter funnel under vacuum into a 500 mL RBF. The collected solution is then reduced under vacuum on a Buchi rotary evaporator to give compound **P.** If a while precipitate is present the crude product is redissolved in hexane and filtered through a plug of 2.54cm (1 inch) of Celite above 2.54cm (1 inch) of flash silica (silica gel 60, EMD) and reduced under vacuum to give Compound **P.**

### Synthesis of Intermediate Q

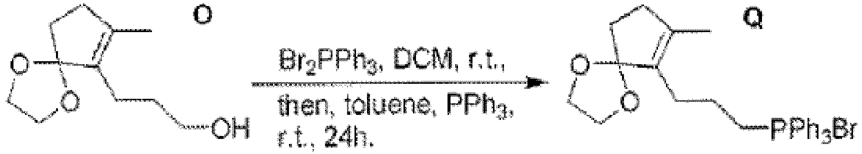

Compound **Q** may prepared by an adaptation of the procedures of Lubell, W. D.; Jamison, T. F.; Rapoport, H.J. Org. Chem., 1990, 55, 3511-3522 and Byrne, P.A.; Gilheany, D. G. J. Am. Chem. Soc., 2012, 134,9225-9239.

To a 500 mL RBF, equipped with a stirrer bar, under an atmosphere of argon at room temperature is added 200 mL of distilled DCM, 9.9 g, 50 mmol of Compound **O** and 42.2 g, 100 mmol of dibromotriphenylphosphorane (Sigma-Aldrich). The reaction mixture is stirred at room temperature and monitored by TLC analysis. An ice bath may be added at the beginning to prevent an exotherm. Once TLC analysis indicates the reaction has gone to completion the reaction mixture is transferred directly to a Buchi rotary evaporator and reduced under vacuum. The residue is taken up in 200 mL of ACS grade toluene and 26.2 g, 100 mmol of triphenylphosphine is added. The reaction mixture is stirred for a further 24 hrs or until completion as indicated by TLC analysis. The reaction mixture is then transferred directly to a Buchi rotary evaporator and reduced under vacuum. The residue is then taken up in a 5:1 mixture of hexane/ethyl acetate and purified by flash column chromatography (Silica gel 60, EMD, hexane/ethyl acetate solvent system) to give Compound **Q.**

### Synthesis of Intermediate R

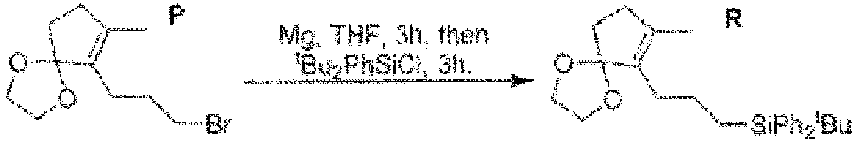

Compound **R** may be prepared by adapting the procedure of Dixon, T. A.; Steele, K. P.; Weber, W. P. J. Organomet. Chem. 1982, 231, 299-305. To a dried 250 mL RBF, equipped with a magnetic stirrer bar, under an argon atmosphere, is added 100 mL of distilled THF, followed by 0.48 g, 20 mmol, of magnesium turnings (Sigma-Aldrich) and the mixture is vigorously stirred at room temperature. Compound **P** (4.96 g, 19 mmol) in 10 mL of distilled THF is the added to the flask via syringe in one portion and the reaction mixture is stirred at room temperature for 3 hours or until most of the magnesium has been consumed, whereupon 4.47 mL, 21 mmol, of tert-butyldiphenylsilyl chloride dissolved in 10 mL of distilled THF is added in one portion via syringe and the reaction is left to stir at room temperature for a further 3 hours. The reaction mixture is subsequently quenched with 50 mL of saturated, aqueous ammonium chloride solution and diluted with 100 mL of ethyl acetate and transferred to separatory funnel. After being vigorously shaken, the biphasic mixture is then separated and the aqueous phase is further extracted with two 50 mL portions of ethyl acetate. The combined organic phases are then dried with sodium sulfate and filtered through a plug of 2.54cm (1 inch) of Celite and 2.54cm (1 inch) of flash silica (silica gel 60, EMD) via a 100 mL sinter funnel under vacuum into a 500 mL rbf, with the sodium sulfate residue washed with a further 50 mL of ethyl acetate. The collected solution is then reduced under vacuum on a Buchi rotary evaporator. The residue is then taken up in a 5:1 mixture of hexane/ethyl acetate and purified by flash column chromatography (Silica gel 60, EMD, hexane/ethyl acetate solvent system) to give Compound **R.**

### Synthesis of Intermediate S

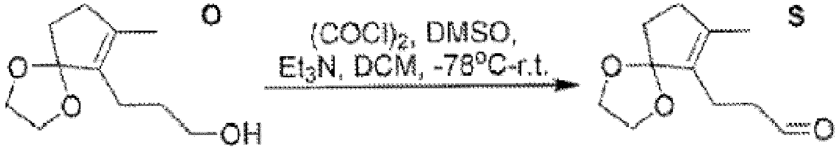

Compound **S** may be prepared by adapting the procedures of Miyata, O.; Muroya, K.; Kobayashi, T.; Yamanaka, R.; Kajisa, S.; Koide, J.; Naito, T. Tetrahedron, 2002, 58, 4459-4479. To a 250 mL RBF, equipped with a stirrer bar, under an atmosphere of argon is added 40 mL of distilled DCM and 0.77 mL, 9 mmol of oxalylchloride (Sigma-Aldrich) and the reaction mixture was cooled to -78°C with a dry ice bath. 1.25 mL, 17.6 mmol of dimethyl sulfoxide (Sigma-Aldrich) is then added drop wise by syringe and the reaction is stirred for a further 10 minutes. After this time a solution of 0.87 g, 4.5 mmol of compound N, dissolved in 10 mL of DCM, is added via syringe and the reaction is stirred for a further 15 minutes whereupon 2.5 mL of triethylamine (Sigma-Aldrich) is added over 5 minutes via syringe. The reaction is stirred for a further 15 minutes before being warmed to 0°C. After TLC analysis shows the reaction is completed the mixture is transferred directly onto a silica gel column (Silica gel 60, EMD) and the Compound S is isolated via flash chromatography (hexane/ethyl acetate solvent system).

### Synthesis of Intermediate T (From Intermediates E and Q)

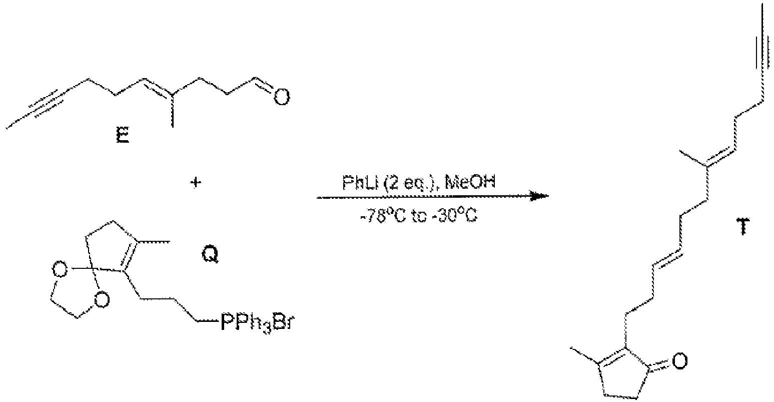

Compound **T** may be prepared by adapting the procedures of Johnson, W. S.; Gravestock, M. B.; McCarry, B. E. J. Am. Chem. Soc., 1971, 93, 4332-4334. To a dried 250 mL RBF under an atmosphere of argon at room temperature is added 100 mL of distilled THF, and 10A4 g, 20 mmol, of Compound **Q.** The resulting solution is then treated with 11.11 mL, 20 mmol, of 1.8 M phenyllithium in dibutyl ether (Sigma-Aldrich) and after 15 minutes is cooled to -78°C with a dry ice bath. After a further 15 minutes, 2.68 g, 20 mmol, of Compound **E** dissolved in 5 mL of dry THF is added via syringe and the reaction mixture is warmed to -30°C through transfer of the apparatus to a cryostat. A second equivalent of 1.8 M phenyllithium is then added followed by excess methanol with the temperature maintained at -30°C. After stirring for 5 minutes the reaction is brought to room temperature and 40 mL of water is added and the reaction mixture is transferred to a 1 L separatory funnel where 200 mL of ethyl acetate is added. After being vigorously shaken, the biphasic mixture is separated and the aqueous phase is further extracted with two 50 mL portions of ethyl acetate. The combined organic phases are then dried with sodium sulfate and filtered through a plug of 2.54cm (1 inch) of Celite via a 100 mL sinter funnel under vacuum into a 1 L RBF, with the sodium sulfate residue washed with a further 50 mL of ether. The collected solution is then reduced under vacuum on a Buchi rotary evaporator. The residue is then taken up in a 5:1 mixture of hexane/ethyl acetate and purified by flash column chromatography (Silica gel 60, EMD, hexane/ethyl acetate solvent system) to give Compound **T.**

### Synthesis of Intermediate T (From Intermediates I and S)

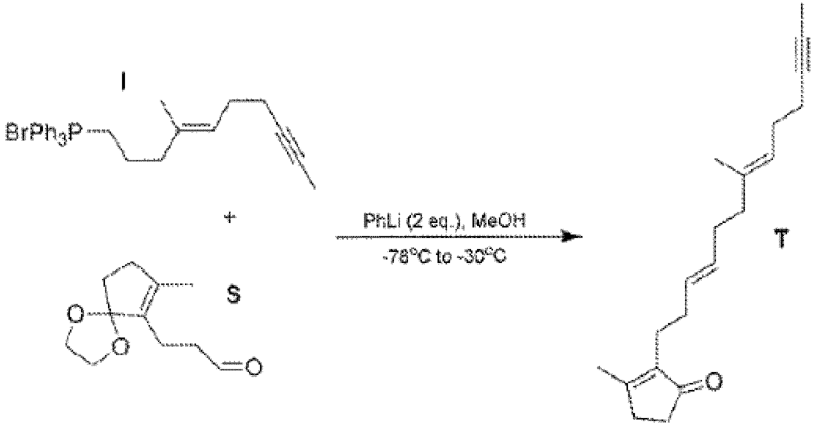

Compound **T** may also be prepared by adapting the procedures of Johnson, W. S.; Gravestock, M. B.; McCarry, B. E. J. Am. Chem. Soc., 1971, 93, 4332-4334 using different starting materials. To a dried 250 mL RBF under an atmosphere of argon at room temperature is added 100 mL of distilled THF, and 7.76 g, 20 mmol, of Compound I. The resulting solution is then treated with 11.11 mL, 20 mmol, of 1.8 M phenyllithium in dibutyl ether (Sigma-Aldrich) and after 15 minutes is cooled to -78°C with a dry ice bath. After a further 15 minutes, 3.92 g, 20 mmol, of Compound S dissolved in 5 mL of dry THF is added via syringe and the reaction mixture is warmed to -30°C through transfer of the apparatus to a cryostat. A second equivalent of 1.8 M phenyllithium is then added followed by excess methanol with the temperature maintained at -30°C. After stirring for 5 minutes the reaction is brought to room temperature and 40 mL of water is added and the reaction mixture is transferred to a 1 L separatory funnel where 200 mL of ethyl acetate is added. After being vigorously shaken, the biphasic mixture is separated and the aqueous phase is further extracted with two 50 mL portions of ethyl acetate. The combined organic phases are then dried with sodium sulfate and filtered through a plug of 2.54cm (1 inch) of Celite via a 100 mL sinter funnel under vacuum into a 1 L RBF, with the sodium sulfate residue washed with a further 50 mL of ether. The collected solution is then reduced under vacuum on a Buchi rotary evaporator. The residue is then taken up in a 5:1 mixture of hexane/ethyl acetate and purified by flash column chromatography (Silica gel 60, EMD, hexane/ethyl acetate solvent system) to give Compound **T.**

### Synthesis of Intermediate T (From Intermediates H and S)

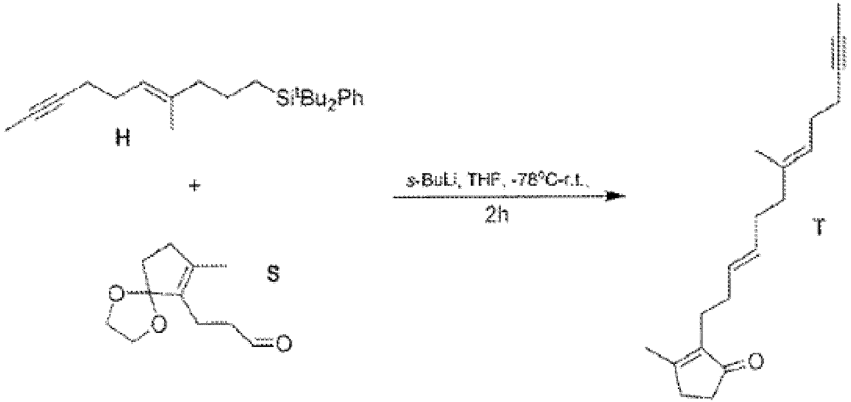

Compound **T** may be prepared by adapting the procedures of W. Adam, C. M. Ortega-Schulte, Synlett, 2003, 414-416 and A. Barbero, Y. Blanco, C. Garcia, Synthesis, 2000, 1223-1228. To a dried 250 mL RBF under an atmosphere of argon at room temperature is added 100 mL of distilled THF, and 9.82 g, 20 mmol, of Compound **H.** The resulting solution is then cooled to -78°C with a dry ice bath and 14.29 mL, 20 mmol, of 1.4 M sec-butyllithium in cyclohexane (Sigma-Aldrich) is added over 5 minutes. After a further 45 minutes, 3.92 g, 20 mmol, of Compound **S** dissolved in 5 mL of dry THF is added via syringe and the reaction mixture is warmed to room temperature After stirring for an additional 2 hours the reaction is diluted with 150 mL of ether and 40 mL of water is then added and the reaction mixture is transferred to a 1 L separatory funnel. After being vigorously shaken, the biphasic mixture is separated and the aqueous phase is further extracted with two 50 mL portions of ether. The combined organic phases are then dried with sodium sulfate and filtered through a plug of 2.54cm (1 inch) of Celite via a 100 mL sinter funnel under vacuum into a 1 L RBF, with the sodium sulfate residue washed with a further 50 mL of ether. The collected solution is then reduced under vacuum on a Buchi rotary evaporator. The residue is then taken up in a 5:1 mixture of hexane/ethyl acetate and purified by flash column chromatography (Silica gel 60, EMD, hexane/ethyl acetate solvent system) to give Compound **T.**

### Synthesis at Intermediate T (From Intermediates E and R)

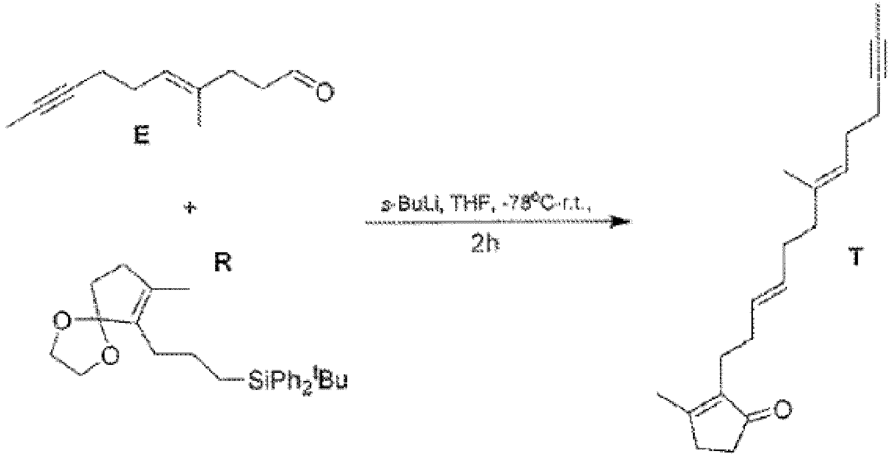

Compound **T** may be prepared by adapting the procedures of W. Adam, C. M. Ortega-Schulte, Synlett, 2003, 414-416 and A. Barbero, Y. Blanco, C. Garcia, Synthesis, 2000, 1223-1228, with different starting materials. To a dried 250 mL RBF under an atmosphere of argon at room temperature is added 100 mL of distilled THF, and 8.4 g, 20 mmol, of Compound **R.** The resulting solution is then cooled to -78°C with a dry ice bath and 14.29 mL, 20 mmol, of 1.4 M sec-butyllithium in cyclohexane (Sigma-Aldrich) is added over 5 minutes. After a further 45 minutes, 2.68 g, 20 mmol, of Compound **E** dissolved in 5 mL of dry THF is added via syringe and the reaction mixture is warmed to room temperature After stirring for an additional 2 hours the reaction is diluted with 150 mL of ether and 40 mL of water is then added and the reaction mixture is transferred to a 1 L separatory funnel. After being vigorously shaken, the biphasic mixture is separated and the aqueous phase is further extracted with two 50 mL portions of ether. The combined organic phases are then dried with sodium sulfate and filtered through a plug of 2.54cm (1 inch) of Celite via a 100 mL sinter funnel under vacuum into a 1 L RBF, with the sodium sulfate residue washed with a further 50 mL of ether. The collected solution is then reduced under vacuum on a Buchi rotary evaporator. The residue is then taken up in a 5:1 mixture of hexane/ethyl acetate and purified by flash column chromatography (Silica gel 60, EMD, hexane/ethyl acetate solvent system) to give Compound **T.**

### Synthesis of Intermediate T (From Intermediates I and Y)

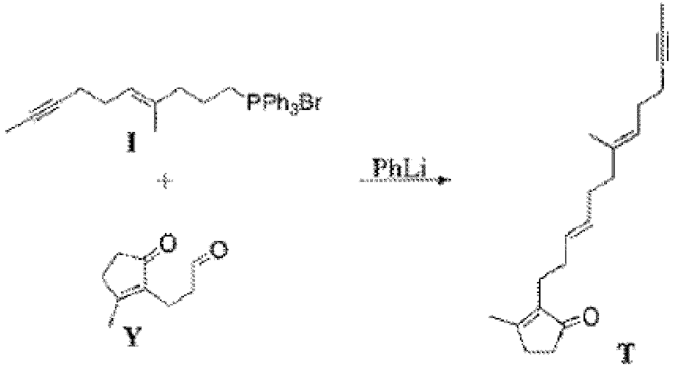

Compound **T** may also be prepared by adapting the procedures of Johnson, W. S.; Gravestock, M. B.; McCarry, B. E. J. Am. Chem. Soc., 1971, 93, 4332-4334 using different starting materials. To a dried 250 mL RBF under an atmosphere of argon at room temperature is added 100 mL of distilled THF, and 7.76 g, 20 mmol, of Compound **I.** The resulting solution is then treated with 11.11 mL, 20 mmol, of 1.8 M phenyllithium in dibutyl ether (Sigma-Aldrich) and after 15 minutes is cooled to -78°C with a dry ice bath. After a further 15 minutes, 20 mmol, of Compound **Y** dissolved in 5 mL of dry THF is added via syringe and the reaction mixture is warmed to -30°C through transfer of the apparatus to a cryostat. A second equivalent of 1.8 M phenyllithium is then added followed by excess methanol with the temperature maintained at - 30°C. After stirring for 5 minutes the reaction is brought to room temperature and 40 mL of water is added and the reaction mixture is transferred to a 1 L separatory funnel where 200 mL of ethyl acetate is added. After being vigorously shaken, the biphasic mixture is separated and the aqueous phase is further extracted with two 50 mL portions of ethyl acetate. The combined organic phases are then dried with sodium sulfate and filtered through a plug of 2.54cm (1 inch) of Celite via a 100 mL sinter funnel under vacuum into a 1 L RBF, with the sodium sulfate residue washed with a further 50 mL of ether. The collected solution is then reduced under vacuum on a Buchi rotary evaporator. The residue is then taken up in a 5:1 mixture of hexane/ethyl acetate and purified by flash column chromatography (Silica gel 60, EMD, hexane/ethyl acetate solvent system) to give Compound **T.**

### Synthesis of Intermediate U|

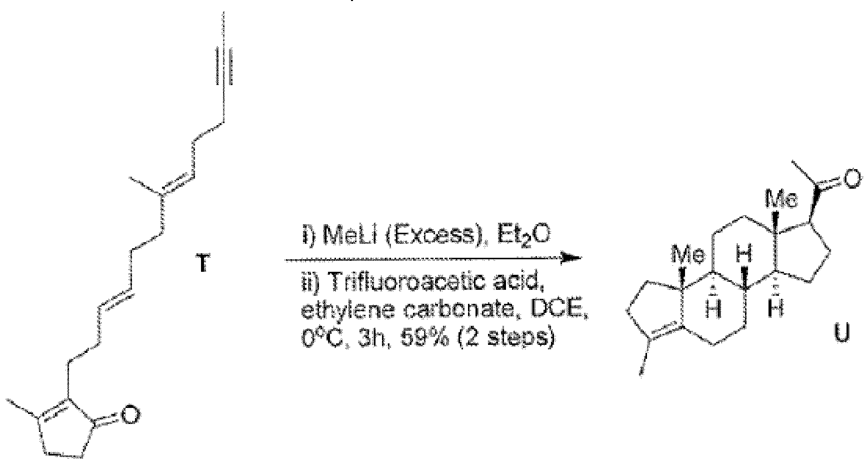

Compound U may be prepared in racemic form by adapting the procedures of Johnson, W. S.; Gravestock, M. B.; McGarry, B. E. J. Am. Chem. Soc., 1971, 93, 4332,4334. To a dried 250 mL RBF, equipped with a stirrer bar, under an atmosphere of argon at room temperature is added 100 mL of distilled ether and 5.68 g, 20 mmol, of Compound T. The resulting solution is then treated with 25 mL, 40 mmol, of 1.6 M methyllithium in ether (Sigma-Aldrich) at room temperature and the reaction mixture monitored by TLC. Once all the starting material is consumed the reaction is quenched with 25 mL of aqueous saturated ammonium chloride and transferred to a 1 L separatory funnel where an additional 200 mL of ether is added. After being vigorously shaken, the biphasic mixture is separated and the aqueous phase is further extracted with two 50 mL portions of ether. The combined organic phases are then dried with sodium sulfate and filtered through a plug of 2.54cm (1 inch) of Celite via a 100 mL sinter funnel under vacuum into a 1 L RBF, with the sodium sulfate residue washed with a further 50 mL of ether. The collected solution is then reduced under vacuum on a Buchi rotary evaporator and the crude alcohol used without further purification due to instability.

Thus in a 500 mL RBF, equipped with a stirrer bar, under argon, the crude alcohol is taken up in 200 mL of distilled dichloroethane (DCE), to which is added 59.5 g of ethylene carbonate. This mixture is then cooled to 0°C with an ice bath and 37 mL of trifluoroacetic acid is added via syringe. The reaction mixture is stirred for 3 hours before excess potassium carbonate in aqueous methanol (50 mL) is added and the reaction mixture is transferred to a 1 L separatory funnel. After being vigorously shaken, the biphasic mixture is separated and the aqueous phase is further extracted with two 50 mL portions of ether. The combined organic phases are then dried with sodium sulfate and filtered through a plug of 2.54cm (1 inch) of Celite via a 100 mL sinter funnel under vacuum into a 1 L RBF, with the sodium sulfate residue washed with a further 50 mL of ether. The collected solution is then reduced under vacuum on a Buchi rotary evaporator. The residue is then taken up in a 5:1 mixture of hexane/ethyl acetate and purified by flash column chromatography (Silica gel 60, EMD, hexane/ethyl acetate solvent system) to give Compound U as a racemic mixture. The enantiomers of compound **U** can be separated to provide enantiomerically-enriched Compound U for use in further synthesis of enantiomerically-enriched ent-progesterone.

### Synthesis of ent-progesterone (From Intermediate U)

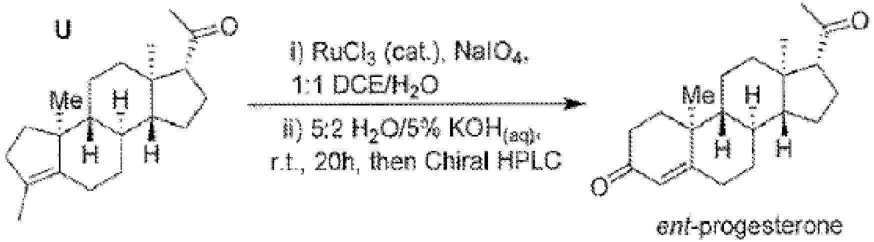

Step I may be prepared by adapting the procedures of Yang, D.; Zhang, C. J. Org. Chem., 2001, 66, 4814-4818; Step ii may be prepared by adapting the procedures of Johnson, W. S.; Gravestock, M. B.; McCarry, B. E. J. Am. Chem. Soc., 1971, 93, 4332-4334. Not in accordance with the present invention and to a 250 mL RBF, equipped with a stirrer bar, under an atmosphere of argon at room temperature is added 100 mL of a 1:1 mixture of DCE/H₂0, 5.68 g, 20 mmol, of Compound **U,** 0.145 g, 0.7 mmol of ruthenium(Ill)chloride (Sigma-Aldrich) and 8.56 g, 40 mmol of sodium periodate (Sigma- Aldrich). The reaction mixture is stirred at room temperature and monitored by TLC. Upon completion the reaction mixture is diluted with 100 mL of ether and transferred to a 500 mL separatory funnel where an additional. After being vigorously shaken, the biphasic mixture is separated and the aqueous phase is further extracted with two 50 mL portions of ether. The combined organic phases are then dried with sodium sulfate and filtered through a plug of 2.54cm (1 inch) of Celite above 2.54cm (1 inch) of flash silica (silica gel 60, EMD) via a 100 mL sinter funnel under vacuum into a 1 L RBF, with the sodium sulfate residue washed with a further 50 mL of ether. The collected solution is then reduced under vacuum on a Buchi rotary evaporator and the crude triketone used without further purification. Thus in a 100 mL RBF, equipped with a stirrer bar, under argon, the crude triketone is treated with 50 mL of 5:2 water/5% potassium hydroxide solution for 20 hours at room temperature. After which time 100 mL of ethyl acetate is added to the reaction mixture, which is then transferred to a 1 L separatory funnel. After being vigorously shaken, the biphasic mixture is separated and the aqueous phase is further extracted with two 50 mL portions of ethyl acetate. The combined organic phases are then dried with sodium sulfate and filtered through a plug of 2.54cm (1 inch) of Celite via a 100 mL sinter funnel under vacuum into a 1 L RBF, with the sodium sulfate residue washed with a further 50 mL of ethyl acetate. The collected solution is then reduced under vacuum on a Buchi rotary evaporator. The residue is then taken up in a 5:1 mixture of hexane/ethyl acetate and purified by flash column chromatography (Silica gel 60, EMD, hexane/ethyl acetate solvent system) to give *rac-*progesterone. The enantiomers are subsequently separated with chiral HPLC to give ent-progesterone.

### Synthesis of Intermediate V

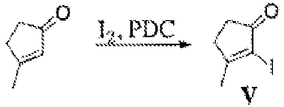

Compound V was prepared according to the method of Richter, A; Hedberg, C; Waldmann, H. J. Org. Chem. 2011, 76, 6694-6702. To a dried round-bottom flask, equipped with a stir bar, under an atmosphere of Argon, was added 400 mL of DCM, 25 mL of 3-methyl-2-cyclopentenone, 95.25 g of iodine, and 28.5 g of pyridinium dichromate sequentially. The reaction was stirred at room temperature for 72-96 h before being washed with a saturated aqueous solution of sodium bisulfite (3 x 200 mL) and reduced under vacuum. The resulting light brown crystalline solid was isolated in 85% yield and used without further purification. ¹H NMR agreed with previously reported data. **¹H NMR (300 MHz, CDCI3): δ = 2.75 (m, 2H), 2.59 (m, 2H), 2.22** (s, **3H).**

### Synthesis of Intermediate W

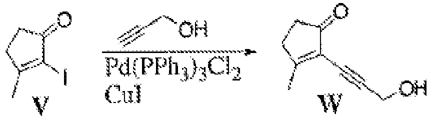

Compound **W** was prepared according to the method of Davie, C. P.; Danheiser, R. L. Angew. Chem. Int. Ed. 2005, 44, 5867-5870. The order of addition of the reagents was altered from the published method. To a dried RBF, equipped with a stir bar, under an atmosphere of Argon, was added 450 mL of THF, 33.6 g of 2-iodo-3-methyl-2-cyclopentenone (compound V), 150 mL of diisopropylamine and 13.468 mL of propargyl alcohol. The mixture was then flushed with argon for 15 mins before 5.34 g of Bis(triphenylphosphine) palladium (II) dichloride and 1.14 g of copper iodide were added sequentially. The reaction mixture was then stirred for 2 h at room temperature whereupon 1H NMR of a small aliquot of the reaction mixture indicated that the reaction had gone to completion. After addition of 250 mL of water the mixture was extracted with DCM (2 x 250 mL) and partially reduced under vacuum until approximately 200 mL of solvent remained. A ¹H NMR of the solution indicated a solvent ratio of approximately 4:4:1 of DCM/THF/'Pr2NH. This mixture was used directly in the preparation of compound **X.** For isolated compound **W - ¹H NMR (300 MHz, CDCI₃): δ = 4.45 (s, 2H), 2.65 (m, 2H), 2.50 (m, 2H), 2.22 (s, 3H), 2J0 (bs, 1H).**

### Synthesis of Intermediate X

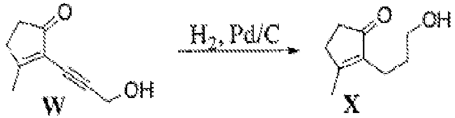

To the isolated mixture described in the preparation of compound W, was added 24 g of 10% Pd on activated carbon (Sigma Aldrich). The reaction flask and mixture where flushed with H2 and subsequently maintained under 1 atmosphere of H2 for 12-24 h. After 1H NMR of the reaction mixture had indicated complete reduction of the intermediate propargyl alcohol the reaction mixture was filtered through celite and reduced under vacuum to give a dark brown/black oil. Further subjection to a short column gave the desired alcohol as a red/brown oil in 70% yield >80% purity. **¹H NMR (300 MHz, CDCI3): δ = 3.52 (**t, **2H), 2.57 (m, 2H), 2.50 (bs, 1H), 2.44 (m, 2H), 2.35 (t, 2H), 2.10 (s, 3H), 1.65 (m, 2H).**

### Synthesis of Intermediate Y

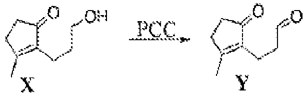

At room temperature 154 mg of the alcohol was dissolved in 10 mL of DCM in a RBF, equipped with a stirrer bar and under an atmosphere of Argon. To this solution was added 431 mg of Celite followed by 431 mg of pyridinium chlorochromate. The reaction mixture was stirred for 4 h whereupon TLC analysis indicated that the reaction had gone to completion. The reaction mixture was reduced under vacuum and the resulting residue extracted with 5:1 hexane/ethyl acetate (4 x 20 mL). The combined extracts were filtered through a plug of silica and reduced under vacuum to give the desired aldehyde as a yellow oil in 74% yield, >85% purity.

### Reference List

1. US Publication No. US 2005/0187188 to Stein et al., published August 25, 2005-"Methods for the Treatment of a Traumatic Central Nervous System Injury"
2. US Publication No. US 2007/0078117 to Hoffman et al., published April 5, 2007-"Methods for the Treatment of a Traumatic Central Nervous System Injury"
3. US Publication No. US 2008/0318914 to Hoffman et al., published December 25, 2008-"Methods for the Treatment of a Traumatic Central Nervous System Injury"
4. US Publication No. US 2009/0221544 to Stein et al., published September 3, 2009-"Methods for the Treatment of a Traumatic Central Nervous System Injury via a Tapered Administration Protocol"
5. US Publication No. US 2009/0325920 to Hoffman et al., published December 31, 2009-"Methods for the Treatment of a Traumatic Central Nervous System Injury"
6. US Publication No. US 2011/0306579 to Stein et al., published December 15, 2011-"Methods of Neuroprotection using Neuroprotective Steroids and a Vitamin D"
7. R. J. Auchus et al. "The Enantiomer of Progesterone (ent-progesterone) is a Competitive Inhibitor of Human Cytochromees P450c17 and P450c21," Archives of Biochemistry and Biophysics 409 (2003) 134-144
8. S. D. Rychnovsky et al. "Synthesis of ent-Cholesterol, the Unnatural Enantiomer," J. Org. Chem. 57 (1992) 2732-2736
9. S. Talengaonkar et al. "Intranasal Delivery: An Approach to Bypass the Blood Brain Barrier," Indian J. Pharmacol, Vol 36, Issue 3, (2004), 140-147
10. H. Nemoto et al. " First Enantioselective Total Synthesis of (+)-Cortisone," J. Org. Chem. 55 (1990) 5625-5631
11. W. S. Johnson et al. "Synthesis of dl-Progesterone," Journal of the American Chemical Society, Vol 93, Issue 17, (1971) 4332-4334
12. M. Weimar et al. "Enantioselective Synthesis of (+)-Estrone Exploiting a Hydrogen Bond-Promoted Diels-Alder Reaction," J. Org. Chem 75 (2010) 2718-2721
13. Herrmann et al. "Formal Total Synthesis of (±)-Estrone and Zirconocene-Promoted Cyclization of 2-Fluoro-1-7-octadienes and Ru-Catalyzed Ring Closing Metathesis," J. Org. Chem 73 (2008) 6202-6206
14. Q. Hu et al. "Simple, Catalytic Enantioselective Synthesis of Estrone and Desogestrel," J. Am Chem Soc 126 (2004) 5984-5986
15. Y. Horiguchi et al. "Total Synthesis of (±)-Cortisone. Double Hydroxylation Reaction for the Construction of Corticoid Side Chain," J. Org. Chem. 51 (1986) 4323¬4325
16. US Patent Application No. 13/645,854 to VanLandingham et al. "Prophylactic and Post-Acute Use of Progesterone to Better Outcomes Associates with Concussion"
17. US Patent Application No. 13/645,881 to VanLandingham et al. "Nasal Delivery Mechanism for Prophylactic and Post-Acute Use for Progesterone and/or Its Enantiomer for Use in Treatment of Mild Traumatic Brain Injuries"
18. US Patent Application No. 13/645,925 to VanLandingham et al. "Prophylactic and Post-Acute Use of Progesterone in Conjunction with its Enantiomer for Use in Treatment of Mild Traumatic Brain Injuries"
19. International PCT Patent Application No. PCT/US2012/59030 to VanLandingham et al. "Prophylactic and Post-Acute Use of Progesterone to Better Outcomes Associated with Concussion"
20. International PCT Patent Application No. PCT/US2012/59087 to VanLandingham et al. "Nasal Delivery Mechanism for Prophylactic and Post-Acute Use for Progesterone and/or Its Enantiomer for Use in Treatment of Mild Traumatic Brain Injuries"
21. International PCT Patent Application No. PCT/US2012/59083 to VanLandingham et al. "Prophylactic and Post-Acute Use of Progesterone in Conjunction with its Enantiomer for Use in Treatment of Mild Traumatic Brain Injuries"
22. Anand Kumar, T.C., David, G.F.X., Sankaranarayanan, A., Puri, V. and Sundram, K. R., 1982. Pharmacokinetics of progesterone after its administration to ovariectomized rhesus monkeys by injection, infusion or intranasal spraying. Proceedings of the National Academy of Science 79, 4185-4189.
23. Anand Kumar, T.C., David, G.F.X., Kumar, K., Umberkoman, B. and Krishnamoorthy, M.S., 1974b. A new approach to fertility regulation by interfering with neuroendocrine pathways. In: Neuroendocrine Regulation of Fertility. Karger, Basel, 314-322.
24. Anand Kumar, T.C., David, G.F., Umberkoman, B. and Saini, K.D., 1974a. Uptake of radioactivity by body fluids and tissues in rhesus monkeys after intravenous injection or intranasal spray of tritium-labelled oestradiol and progesterone. Curr. Sci. 43, 435439.
25. Cincinelli et al., "Effects of the repetitive administration of progesterone by nasal spray in postmenopausal women," Fertil. Steril. 60(6): 1020-1024 (1993).

## Claims

1. A method for preparing ent-progesterone, the method comprising the step
of (a) reacting a compound of the formula **U:** 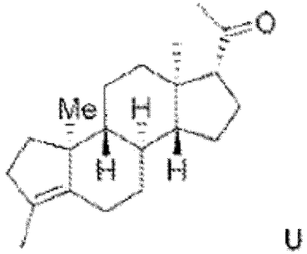
to form a triketone intermediate of the formula **U':** 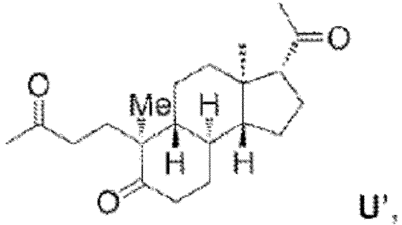
via the steps of:
i. contacting the compound of formula U with a dihydroxylating reagent which is an osmium reagent or a manganese reagent, thereby forming a vicinal diol, and,
ii. oxidatively cleaving the vicinal diol, thereby forming the compound of formula **U',** and
(b) cyclizing the compound of formula **U'** to form ent-progesterone.

2. The method of claim 1, wherein the dihydroxylating reagent comprises an osmium catalyst, a manganese catalyst, and the step of oxidatively cleaving the vicinal diol comprises contacting the vicinal diol with a periodate reagent or lead tetraacetate.

## Patentansprüche

1. Verfahren zum Herstellen von Ent-Progesteron, wobei das Verfahren Folgendes umfasst:
den Schritt (a) Umsetzen einer Verbindung der Formel U: 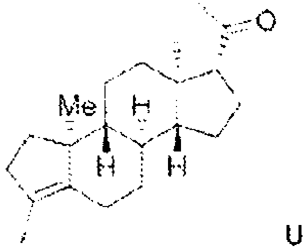
um ein Triketon-Zwischenprodukt der Formel U' auszubilden: 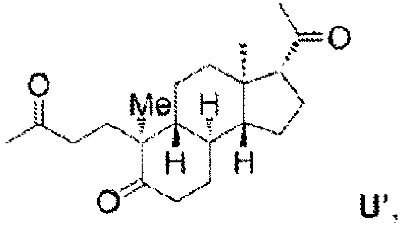
über die folgenden Schritte:
i. Inberührungbringen der Verbindung der Formel U mit einem Dihydroxylierungsreagens, das ein Osmiumreagens oder ein Manganreagens ist, wobei dadurch ein vicinales Diol ausgebildet wird, und
ii. oxidatives Spalten des vicinalen Diols, wobei dadurch die Verbindung der Formel U' ausgebildet wird, und
(b) Cyclisieren der Verbindung der Formel U', um Ent-Progesteron auszubilden.

2. Verfahren nach Anspruch 1, wobei das Dihydroxylierungsreagens einen Osmiumkatalysator, einen Mangankatalysator umfasst und der Schritt des oxidativen Spaltens des vicinalen Diols das Inberührungbringen des vicinalen Diols mit einem Periodatreagens oder einem Bleitetraacetat umfasst.

## Revendications

1. Procédé destiné à la préparation d'ent-progestérone, le procédé comprenant l'étape de
(a) réaction d'un composé de formule **U** : 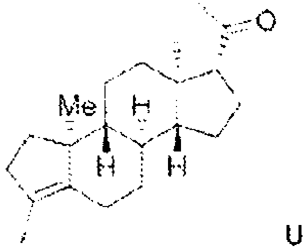
pour former un intermédiaire tricétone de formule **U'** : 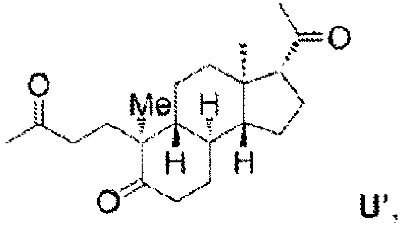
par l'intermédiaire des étapes de :
i. mise en contact du composé de formule U avec un réactif de dihydroxylation qui est un réactif d'osmium ou un réactif de manganèse, formant ainsi un diol vicinal, et,
ii. clivage par oxydation du diol vicinal, formant ainsi le composé de formule **U',** et
(b) cyclisation du composé de formule **U'** pour former l'ent-progestérone.

2. Procédé selon la revendication 1, dans lequel le réactif de dihydroxylation comprend un catalyseur à base d'osmium, un catalyseur à base de manganèse, et l'étape de clivage par oxydation du diol vicinal comprend la mise en contact du diol vicinal avec un réactif de periodate ou du tétraacétate de plomb.
